# EUROPEAN PATENT APPLICATION

(11) **EP 1 693 365 A1**
(43) Date of publication of application: **23.08.2006**
(21) Application number: 06075990.9
(22) Date of filing: 26.04.2001
(51) Int. Cl.: C07D 207/16, C07D 277/06, C07D 295/18, C07D 401/12, C07D 403/12, C07D 405/12, C07D 409/12, C07D 417/06, C07D 413/12, C07D 417/12, C07D 471/04, A61K 31/425, A61P 5/50

(54) **Inhibitors of dipeptidyl peptidase IV**

(30) Priority: 26.04.2000 GB 0010183
(62) Divisional of application: 01923861.7
(71) Applicant: Ferring B.V., 2132 JX Hoofddorp (NL)
(72) Inventor: Evans, David Michael, St. Denys Southampton SO17 2HX (GB); Pitt, Gary Robert William, Tidworth, Hampshire SP9 7US (GB)
(74) Representative: Bates, Philip Ian

(57) **Abstract**

Compounds according to general formula 1, wherein R¹ is H or CN, X¹ is S, O, SO₂ or CH₂, X² is O, S or CH₂, X³ is NR⁵ or a carbonyl or thiocarbonyl group and R⁴ is R⁶R⁷N, R⁸(CH₂)_{q}OC(=O), R⁸(CH₂)_{q}OC(=S), R⁶R⁷NC(=O), R⁶R⁷NC(=S); R⁸(CH₂)_{q}C(=O), R⁸(CH₂)_{q}C(=S) or R⁸(CH₂)_{q}SO₂, m is 1-3 and n is 0-4 are new. The compounds of the invention are inhibitors of dipeptidyl peptidase IV. Pharmaceutical compositions of the compounds of the invention, or pharmaceutically acceptable salts thereof, are useful in the treatment of, *inter alia,* type 2 diabetes.

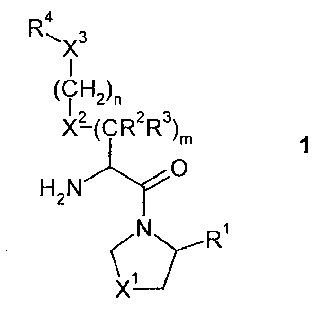

## Description

The present invention relates to a series of novel compounds that are inhibitors of the enzyme dipeptidyl peptidase IV, to pharmaceutical compositions comprising these inhibitors, and the use of such compositions in the treatment of human diseases.

### Background

The enzyme dipeptidyl peptidase IV, herein abbreviated DP-IV (and elsewhere as DAP-IV or DPP-IV) and also known by the classification EC.3.4.14.5, is a serine protease that cleaves the N-terminal dipeptide from peptides that begin with the sequence H-Xaa-Pro (where Xaa is any amino acid, although preferably a lipophilic one, and Pro is proline). It will also accept as substrates peptides that begin with the sequence H-Xaa-Ala (where Ala is alanine). DP-IV was first identified as a membrane-bound protein. More recently a soluble form has been identified.

Initial interest in DP-IV focussed on its role in the activation of T lymphocytes. DP-IV is identical to the T cell protein CD26. It was proposed that inhibitors of DP-IV would be capable of modulating T cell responsiveness, and so could be developed as novel immunomodulators. It was further suggested that CD26 was a necessary co-receptor for HIV, and thus that DP-IV inhibitors could be useful in the treatment of AIDS.

Attention was given to the role of DP-IV outside the immune system. It was recognised that DP-IV has a key role in the degradation of several peptide hormones, including growth hormone releasing hormone (GHRH) and glucagon-like peptide-1 and -2 (GLP-1 and GLP-2). Since GLP-1 is known to have a potentiating effect on the action of insulin in the control of post-prandial blood glucose levels it is clear that DP-IV inhibitors might also be usefully employed in the treatment of type II diabetes and impaired glucose tolerance. At least two DP-IV inhibitors are currently undergoing clinical trials to explore this possibility.

Several groups have disclosed inhibitors of DP-IV. While some leads have been found from random screening programs, the majority of the work in this field has been directed towards the investigation of substrate analogs. Inhibitors of DP-IV that are substrate analogs are disclosed in, for example, US 5,462,928, US 5,543,396, WO95/15309 (equivalent to US 5,939,560 and EP 0731789), WO98/19998 (equivalent to US 6,011,155), WO99/46272 and WO99/61431. The most potent inhibitors are aminoacyl pyrrolidine boronic acids, but these are unstable and tend to cyclise, while the more stable pyrrolidine and thiazolidine derivatives have a lower affinity for the enzyme and so would require large doses in a clinical situation. Pyrrolidine nitriles appear to offer a good compromise since they have both a high affinity for the enzyme and a reasonably long half-life in solution as the free base. There remains, however, a need for inhibitors of DP-IV with improved properties.

### Brief Description of the Invention

The present invention relates to a series of inhibitors of DP-IV with improved affinity for the enzyme. The compounds can be used for the treatment of a number of human diseases, including impaired glucose tolerance and type II diabetes. Accordingly, the invention further relates to the use of the compounds in the preparation of pharmaceutical compositions, to such compositions *per se,* and to the use of such compositions in human therapy. The compounds of the invention are described by general formula **1**.

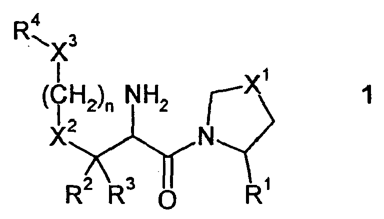

In general formula **1**, X¹ is selected from -S-, -O-, -SO-, -SO₂- and -CH₂-; X² is selected from -O-, -S-, -NH- and -CH₂-; X³ is either -NR⁵- or a >C=O or >C=S group; R¹ is either H or -CN; R² and R³ are independently selected from H and lower alkyl, or together may be -(CH₂)ₚ-; R⁴ is R^{4A} when X³ is -NR⁵- and R^{4B} when X³ is >C=O or >C=S; R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=S) and R⁸(CH₂)_{q}OC(=O); R^{4B} is R⁶R⁷N; R⁵ is H or lower alkyl; R⁶ and R⁷ are each independently R⁸(CH₂)_{q} or together they are -(CH₂)₂-Z-(CH₂)₂-; R⁸ is selected from H, alkyl, optionally substituted aryl, optionally substituted aroyl, optionally substituted arylsulphonyl and optionally substituted heteroaryl; Z is selected from a covalent bond, -(CH₂)ᵣ-, -O-, -SOₜ- and -N(CH₂)_{q}R⁸)-; n is 0 - 4; p is 2 - 5; q is 0 - 3; r is 1 or 2; and t is 0 -2.

### Detailed Description of the Invention

In a first aspect, the present invention comprises a series of novel compounds that are inhibitors of the enzyme DP-IV and are useful for the treatment of certain human diseases. The compounds are described by general formula **1**.

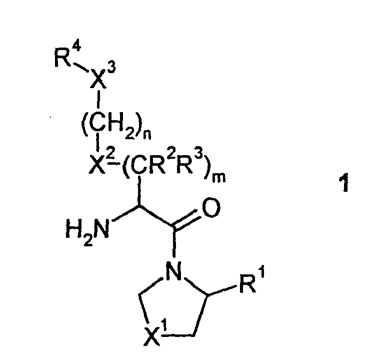

In general formula **1**, X¹ is a divalent group selected from a sulphur atom (-S-), an oxygen atom (-O-), a sulphinyl group (-SO-), a sulphonyl group (-SO₂-) and a methylene group (-CH₂-). X² is a divalent group selected from an oxygen atom (-0-), a sulphur atom (-S-) and a methylene group (-CH₂-). X³ is either a substituted imino group (-NR⁵-) or a carbonyl (>C=O) or thiocarbonyl (>C=S) group.

R¹ is either a hydrogen atom (H) or a nitrile group (-CN).

R² and R³ may each independently of the other be a hydrogen atom or a lower alkyl group, or together they may be a chain of between two and five methylene units (-(CH₂)ₚ- where p is in the range 2 - 5) so as to form, with the carbon atom to which they are attached, a three, four, five or six-membered ring. The value of m may be 1, 2 or 3. When m is greater than 1 then each CR²R³ unit may be the same or different. For example, when m is 2 then (CR²R³)₂ may be CH₂CH₂, CH₂C(Me)₂, C(Me)₂CH₂ and the like.

The nature of R⁴ depends on the identity of X³, such that the two groups are linked by an amide (CO-N), thioamide (CS-N) or sulphonamide (SO₂-N) bond. So, when X³ is a substituted imino group (-NR⁵-) then R⁴ is R^{4A}, where R^{4A} is selected from carbamoyl groups (R⁶R⁷NC(=O)), thiocarbamoyl groups (R⁶R⁷NC(=S)); optionally modified acyl groups (R⁸(CH₂)_{q}C(=O)), optionally modified thioacyl groups (R⁸(CH₂)_{q}C(=S)), sulphonyl groups (R⁸(CH₂)_{q}SO₂), optionally modified (alkyl or aryloxy)carbonyl groups (R⁸(CH₂)_{q}OC(=O)) and optionally modified (alkyl or aryloxy)thiocarbonyl groups(R⁸(CH₂)_{q}OC(=S)). As used herein, the term "optionally modified" is taken to indicate that some embodiments of R⁸ are beyond the scope of the terms "alkyl", "acyl" and "aryl". The scope of the definition of R^{4A} is determined by the scope of the definition of R⁸. Alternatively, when X³ is a carbonyl (>C=O) or thiocarbonyl (>C=S) group then R⁴ is R^{4B}, where R^{4B} is a substituted amino group (R⁶R⁷N).

R⁵ is a hydrogen atom (H) or a lower alkyl group. Preferably, R⁵ is H.

R⁶ and R⁷ may each independently of the other be R⁸(CH₂)_{q}. Alternatively, they may together be a group -(CH₂)₂-Z¹-(CH₂)₂- or -CHR⁹-Z²-CH₂-CR¹⁰-. Here Z¹ is a covalent bond, a methylene or ethylidene group (-(CH₂)ᵣ- where r is 1 or 2), an oxygen atom (-O-), a sulphur or oxidised sulphur atom (-SOₜ- where t is zero, 1 or 2) or a substituted imino group (-N((CH₂)_{q}R⁸)-), such that the group NR⁶R⁷ is a pyrrolidine, piperidine, perhydroazepine, morpholine, optionally oxidised thiomorpholine or substituted piperazine ring. Z² is an *ortho*-phenylene moiety (-C₆H₄-), such that the group NR⁶R⁷ is a tetrahydroisoquinoline.

R⁸ is selected from a hydrogen atom (H), a lower alkyl group, a benzo-fused lower cycloalkyl group (such as an indanyl group), an acyl (lower alkyl-CO) group, a di(lower alkyl)amino group, a di(lower atkyl)carbamoyl group, an N-(lower alkyl)piperidinyl group, an optionally substitiued α-alkylbenzyl group, an optionally substituted phenyl, naphthyl or heteroaryl group, and an optionally substituted aroyl (aryl-CO) or arylsulphonyl (aryl-SO₂) group. In the foregoing, suitable optional substituents are lower alkyl, aryl which may be further substituted with one or more methyl or trifluoromethyl groups, hydroxy, lower alkyloxy, lower alkylsulphonyl, acyl, perfluoroacyl, amino, lower alkylamino, di(lower alkyl)amino, aminoalkylene, fluoro, chloro, bromo, trifluoromethyl, nitro, cyano, carbamoyl, carboxy and lower alkyloxycarbonyl groups. In addition, two adjacent substituents may be linked so as to form a ring fused to the parent aryl or heteroaryl ring.

R⁹ and R¹⁰ are independently selected from hydrogen, carbamoyl, hydroxymethyl and cyanomethyl groups.

The integer n is selected from the range zero to 4, and q is selected from the range zero to 3.

Certain compounds are specifically excluded from the scope of the present invention. When X² is methylene, X³ is NH and R⁴ is R⁸(CH₂)_{q}O(CO), with q = 1, then R⁸ may not be unsubstituted phenyl or phenyl substituted with a nitro group. It is generally preferred that when X² is methylene, X³ is NH, R⁴ is R⁸(CH₂)_{q}O(CO), q is 1 and R⁸ is a substituted phenyl group then the substituent or substituents should be selected from chloro, methoxy and trifluoromethyl groups.

In the context of the present disclosure, the term lower alkyl, either by itself or in such combinations as lower alkyloxy, is intended to comprise linear, branched and cyclic saturated hydrocarbon groups of between one and six carbon atoms. Examples of lower alkyl groups include, but are not limited to, methyl, ethyl, isopropyl, *tert*-butyl, neopentyl, cyclohexyl, cyclopentylmethyl, 2-(cyclopropyl)ethyl, 3,3-dimethylcyclobutyl and bicyclo[3.1.0]hexyl.

The term heteroaryl includes monocyclic five- and six-membered ring aromatic groups with one or two heteroatoms, which are selected from nitrogen, oxygen and sulphur. Thus, heteroaryl groups include pyridyl, pyridazinyl, pyrimidinyl, pyrazinyl, pyrrolyl, thienyl, furyl, imidazolyl, pyrazolyl, thiazolyl, isothiazolyl, oxazolyl and isoxazolyl. Heteroaryl further includes the benzofused derivatives of these rings, such as quinolinyl, isoquinolinyl, quinoxalinyl, quinazolinyl, phthalazinyl, cinnolinyl, indolyl, isoindolyl, benzothiazolyl and the like, and bicyclic groups formed by the fusion of two such monocyclic heteroaromatic groups.

The term aryl includes phenyl, naphthyl and heteroaryl.

The compounds of general formula 1 have at least one stereogenic centre and so can exhibit optical isomerism. All such isomers, including enantiomers, diastereomers and epimers are included within the scope of the invention. Furthermore, the invention includes such compounds as single isomers and as mixtures, including racemates. Certain compounds according to general formula 1, including those with a heteroaryl group which carries a hydroxy or amino substituent, can exist as tautomers. These tautomers, either separately or as mixtures, are also considered to be within the scope of the invention.

The compounds according to general formula 1 have at least one basic functional group. They can therefore form addition salts with acids. Those addition salts that are formed with pharmaceutically acceptable acids are included within the scope of the invention. Examples of suitable acids indude acetic acid, trifluoroacetic acid, citric acid, fumaric acid, benzoic acid, pamoic acid, methanesulphonic acid, hydrochloric acid, nitric acid, sulphuric acid, phosphoric acid and the like.

Certain compounds according to general formula 1 have an acidic group and so are able to form salts with bases. Examples of such salts include the sodium, potassium and calcium salts, which are formed by the reaction of the acid with the corresponding metal hydroxide, oxide, carbonate or bicarbonate. Similarly, tetra-alkyl ammonium salts may be formed by the reaction of the acid with a tetra-alkyl ammonium hydroxide. Primary, secondary and tertiary amines, such as triethylamine, can form addition salts with the acid. A particular case of this would be an internal addition salt formed between an acidic group and the primary amine group of the same molecule, which is also called a zwitterion. Insofar as they are pharmaceutically acceptable, all these salts are included within the scope of the invention.

In a preferred embodiment of the invention R¹ is a nitrile group. Within this embodiment, it is preferred that the stereochemistry of the nitrile group is as shown in general formula **2**.

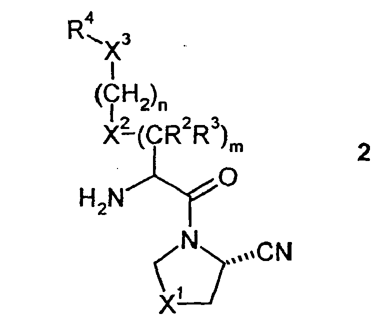

According to the standard terminology, this is the *S* configuration when X¹ is methylene but the *R* configuration when X¹ is sulphur, oxygen, sulphinyl or sulphonyl.

In another preferred embodiment, the stereochemistry at the centre adjacent to the primary amine is as shown in general formula **3**. This is the *S* configuration when X² is an oxygen atom or a methylene or imino group, and the R configuration when X² is a sulphur atom.

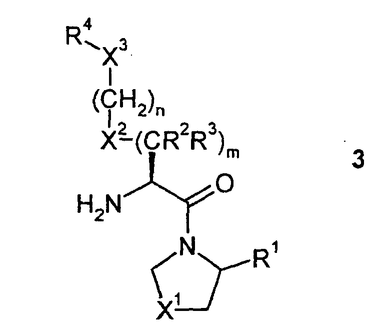

Within this embodiment, it is more preferred that R¹ should be a nitrile group, and more preferred still that it should have the absolute configuration depicted in general formula **4**.

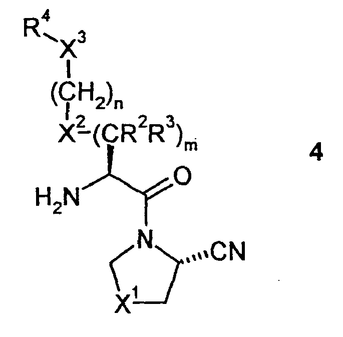

In another preferred embodiment of the invention, m is 1. More preferably m is 1 and R² and R³ are independently hydrogen atoms or methyl groups. When X² is a methylene group, it is more preferred that R² and R³ both be hydrogen. When X² is an oxygen atom, it is more preferred that one of R² and R³ be hydrogen and the other a methyl group. When X² is a sulphur atom, it is more preferred that both R² and R³ be methyl groups.

In another preferred embodiment, X¹ is either S or methylene. More preferably, X¹ is S and R¹ is H, or X¹ is methylene and R¹ is CN.

In another preferred embodiment, X³ is NH. More preferably, X³ is NH, m is 1, R² and R³ are both H, X² is methylene and n is 1 or 2.

In another preferred embodiment, R⁴ is R⁸NHCO or R⁸CO and R⁸ is an optionally substituted heteroaryl group. More preferably, R⁸ is an unsubstituted heteroaryl group, or a heteroaryl group substituted with one or two groups chosen from lower alkyl, lower alkyloxy, fluoro, chloro and trifluoromethyl groups.

In another preferred embodiment, X³ is CO and R⁴ is R⁸NH. More preferably R⁸ is an optionally substituted heteroaryl group. More preferably still, R⁸ is an unsubstituted heteroaryl group, or a heteroaryl group substituted with one or two groups chosen from lower alkyl, lower alkyloxy, fluoro, chloro and trifluoromethyl groups.

In another preferred embodiment, X³ is NH and R⁴ is selected from R⁶R⁷N(CO), R⁸(CH₂)_{q}CO and R⁸(CH₂)_{q}SO₂.

Particularly preferred compounds within the invention include:
(2*S*)-1-[*N*^{ω}-(Pyrazinyl-2-carbonyl)-L-omithinyl]pyrrolidine-2-carbonitrile,
(2*S*)-1-[*N*^{ω}-(Pyrazinyl-2-carbonyl)-L-lysinyl]pyrrolidine-2-carbonitrile,
(2*S*)-1-[(2'*S*)-2'-Amino-4'-(pyrazinyl-2"-carbonylamino)butanoyl]pyrrolidine-2-carbonitrile,
(4*R*)-3-[*N*^{ω}-(Pyrazinyl-2-carbonyl)-L-lysinyl]thiazolidine-4-carbonitrile,
(2*S*)-1-[*N*^{ω}-(Pyridyl-3-methyl)-L-glutaminyl]pyrrolidine-2-carbonitrile,
1-[*N*^{ω}-(Pyrazinyl-2-carbonyl)-L-ornithinyl]pyrrolidine,
(2*S*)-1-[*S*-(Acetylaminomethyl)-L-cysteinyl]pyrrolidine-2-carbonitrile,
3-[*N*^{ω}-(2-Chloropyridyl-3-carbonyl)-L-ornithinyl]thiazolidine,
1-[*N*^{ω}-(2-Chloropyridyl-3-carbonyl)-L-omithinyl]pyrrolidine, and
(2S)-1-[*N*^{ω}-(2-Chloropyridyl-3-carbonyl)-L-ornithinyl]pyrrolidine-2-carbonitrile
3-[N^{ω}-(Pyrazinyl-2-carbonyl)-L-ornithinyl]thiazolidine,
3-[*N*^{ω}-(2-Quinoxaloyl)-L-lysinyl]thiazolidine,
3-[*N*^{ω}-(2-Quinoxaloyl)-L-omithinyl]thiazolidine,
(2S)-1-[*N*^{ω}-(2-Quinoxaloyl)-L-ornithinyl]pyrrolidine-2-carbonitrile,
3-[*N*^{ω}-(6-Methylpyrazinyl-2-carbonyl)-L-ornithinyl]thiazolidine,
3-[*N*^{ω}-(Isoquinoline-3-carbonyl)-L-ornithinyl]thiazolidine,
3-[*N*^{ω}-(6-Trifluoromethylnicotinoyl)-L-ornithinyl]thiazolidine,
(2*S*)-1-[(2'*R*)-3'-(Acetylaminomethylthio)-2'-amino-3'-methylbutanoyl]pyrrolidine-2-carbonitrile,
(2*S*)-1-[*S*-(3-Picolylcarbamoylmethyl)-L-cysteinyl]pyrrolidine-2-carbonitrile,
3-[*N*^{ω}-(3-Pyridyloxycarbonyl)-L-ornithinyl]thiazolidine,
3-[*O*-(3-Chlorobenzylcarbamoyl)serinyl]thiazolidine, and
3-[(2'*S*)-2'-Amino-5'-oxo-5'-(1",2",3",4"-tetrahydroisoquinolin-2"-yl)pentanoyl]thiazolidine.

In a second aspect, the present invention comprises a pharmaceutical composition for human therapeutic use. The composition is characterised in that it has, as an active agent, at least one of the compounds described above. Such a composition is useful in the treatment of human diseases. The composition will generally include one or more additional components selected from pharmaceutically acceptable excipients and pharmaceutically active agents other than those of the present invention.

The composition may be presented as a solid or liquid formulation, depending on the intended route of administration. Examples of solid formulations include pills, tablets, capsules and powders for oral administration, suppositories for rectal or vaginal administration, powders for nasal or pulmonary administration, and patches for transdermal or transmucosal (such as buccal) administration. Examples of liquid formulations include solutions and suspensions for intravenous, subcutaneous or intramuscular injection and oral, nasal or pulmonary administration. A particularly preferred presentation is a tablet for oral administration. Another preferred presentation, particularly for emergency and critical care, is a sterile solution for intravenous injection.

The composition comprises at least one compound according to the preceding description. The composition may contain more than one such compound, but in general it is preferred that it should comprise only one. The amount of the compound used in the composition will be such that the total daily dose of the active agent can be administered in one to four convenient dose units. For example, the composition can be a tablet containing an amount of compound equal to the total daily dose necessary, said tablet to be taken once per day. Alternatively, the tablet can contain half (or one third, or one quarter) of the daily dose, to be taken twice (or three or four times) per day. Such a tablet can also be scored to facilitate divided dosing, so that, for example, a tablet comprising a full daily dose can be broken into half and administered in two portions. Preferably, a tablet or other unit dosage form will contain between 0.1mg and 1g of active compound. More preferably, it will contain between 1 mg and 250mg.

The composition will generally include one or more excipients selected from those that are recognised as being pharmaceutically acceptable. Suitable excipients include, but are not limited to, bulking agents, binding agents, diluents, solvents, preservatives and flavouring agents. Agents that modify the release characteristics of the composition, such as polymers that selectively dissolve in the intestine ("enteric coatings") are also considered in the context of the present invention, to be suitable excipients.

The composition may comprise, in addition to the compound of the invention, a second pharmaceutically active agent. For example, the composition may include an anti-diabetic agent, a growth-promoting agent, an anti-inflammatory agent or an antiviral agent. However, it is generally preferred that the composition comprise only one active agent.

In a third aspect, the invention comprises a use for the compounds and compositions described above for the treatment of human diseases. This aspect can equally be considered to comprise a method of treatment for such diseases. The diseases susceptible to treatment are those wherein an inhibition of DP-IV or CD26 results in a clinical benefit either directly or indirectly. Direct effects include the blockade of T lymphocyte activation. Indirect effects include the potentiation of peptide hormone activity by preventing the degradation of these hormones. Examples of diseases include, but are not limited to, auto-immune and inflammatory diseases such as inflammatory bowel disease and rheumatoid arthritis, growth hormone deficiency leading to short stature, polycystic ovary syndrome, impaired glucose tolerance and type 2 diabetes. Particularly preferred is the use of the compounds and compositions for the treatment of impaired glucose tolerance and type 2 diabetes, and equally a method of treatment of these diseases by the administration of an effective amount of a compound or composition as previously described.

The precise details of the treatment, including the dosing regimen, will be established by the attending physician taking into account the general profile of the patient and the severity of the disease. For diseases such as inflammatory bowel disease that have acute phases of active disease separated by quiescent periods, the physician may select a relatively high dose during the acute phase and a lower maintenance dose for the quiescent period. For chronic diseases such as type 2 diabetes and impaired glucose tolerance, the dosing may need to be maintained at the same level for an extended period. A dosing schedule of one to four tablets per day, each comprising between 0.1mg and 1g (and preferably between 1 mg and 250mg) of active compound might be typical in such a case.

The compounds according to the invention can be prepared by methods known in the art. The route chosen will depend on the particular nature of the substituents present in the target molecule. In the following general description the synthetic route is outlined for compounds wherein m is 1. Compounds with m = 2 or 3 can generally be prepared by analogous routes.

The starting material will usually be an α,ω-diamino acid derivative **5** or an amino dicarboxylic acid derivative **6**.

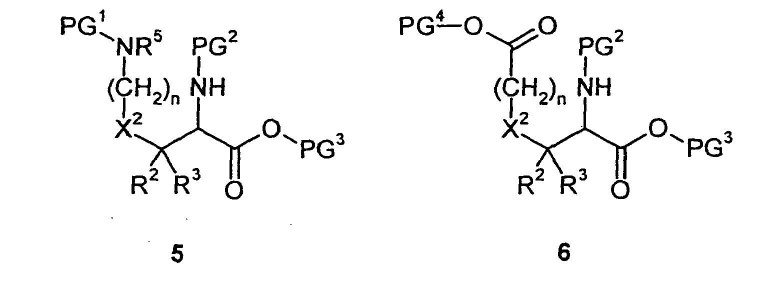

PG¹ and PG² are "orthogonal" protecting groups - groups that mask the reactivity of the amine groups and that can each be selectively removed in the presence of the other. Suitable groups are well known in the literature. PG³ and PG⁴ are protecting groups for carboxylic acids. They are chosen such that they are orthogonal to each other and to the amino protecting groups. Suitable possibilities for PG³ and PG⁴ are also well known in the literature. Derivatives of diamino acids according to general formula **5** and derivatives of amino dicarboxylic acids according to general formula 6 are either items of commerce, or can be prepared following methods described in the literature. In practice, and depending on the strategy chosen, the starting material will have only two of the three protecting groups present. Either PG³ will be absent to allow the pyrrolidine (or thiazolidine or oxazolidine) residue to be introduced, or PG¹ or PG⁴ will be absent to allow the side chain to be elaborated.

Scheme A illustrates the introduction of the pyrrolidine (or thiazolidine or oxazolidine) group as the first step in the preparation of the compounds of the invention.

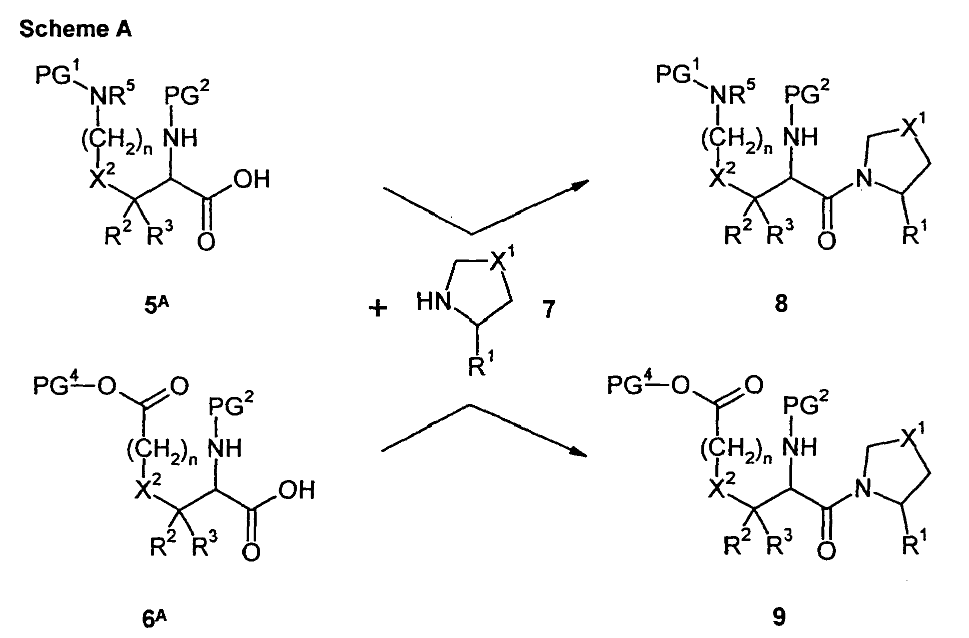

Compounds **5**^{**A**} and **6**^{**A**} correspond to **5** and **6** with PG³ as a hydrogen atom (i.e. without the protecting group). The free carboxylic acid can be reacted with a pyrrolidine derivative **7** to give the amide **8** or **9**. Reaction conditions for achieving this transformation are well known in the literature. Suitable reagents include carbodiimides, phosphorus reagents and alkyl chloroformates, and the reaction is usually catalysed by a tertiary amine such as triethylamine or dimethylaminopyridine.

The reaction depicted in Scheme A is available for all combinations of R¹ and X¹. However, for the case where R¹ is a nitrile group, or where X¹ is a sulphinyl or sulphonyl group, it may be advantageous to modify the strategy as depicted in Schemes B and C.

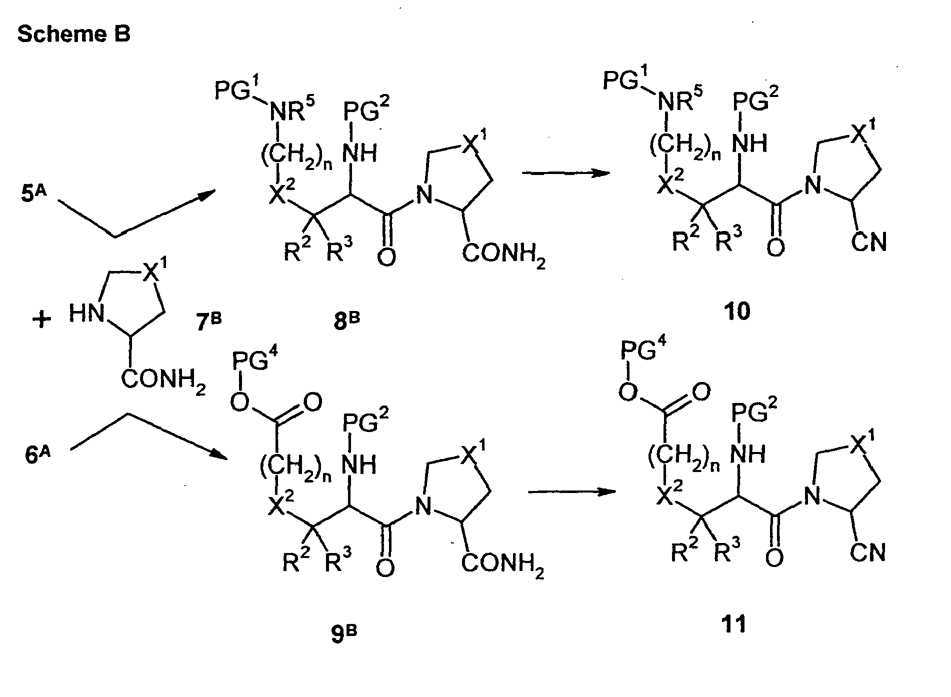

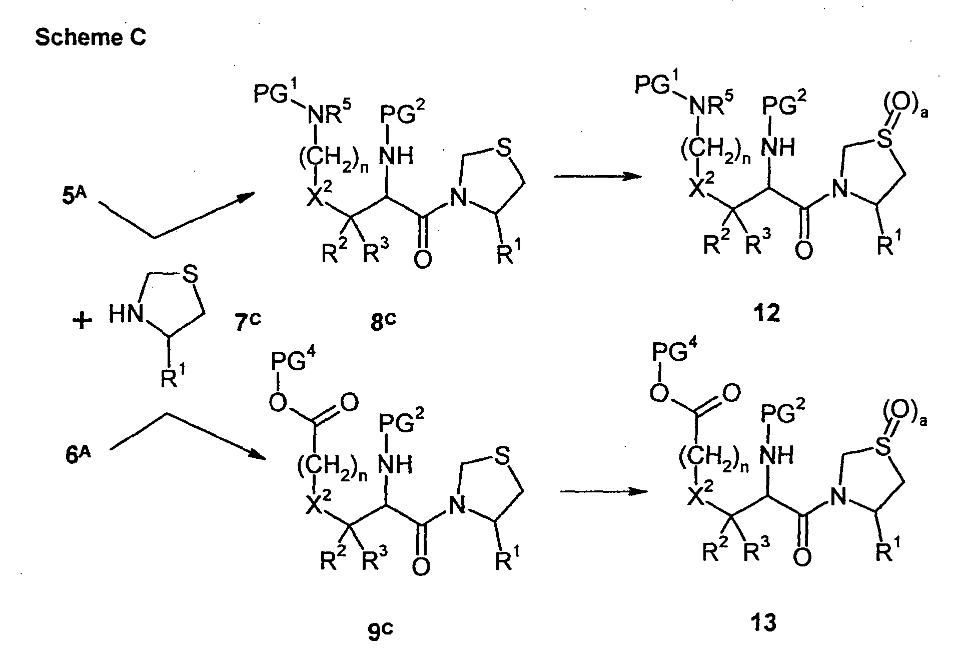

In Scheme B, the R¹ group is introduced as a primary amide and subsequently transformed into a nitrile by the action of a dehydrating agent such as trifluoroacetic anhydride. In Scheme C, the X¹ group is introduced as a thioether and subsequently transformed into a sulphoxide (a=1) or sulphone (a=2) by the action of an oxidant such as sodium periodate. The modification to the strategy afforded by Scheme C is not possible if X² is a sulphur atom.

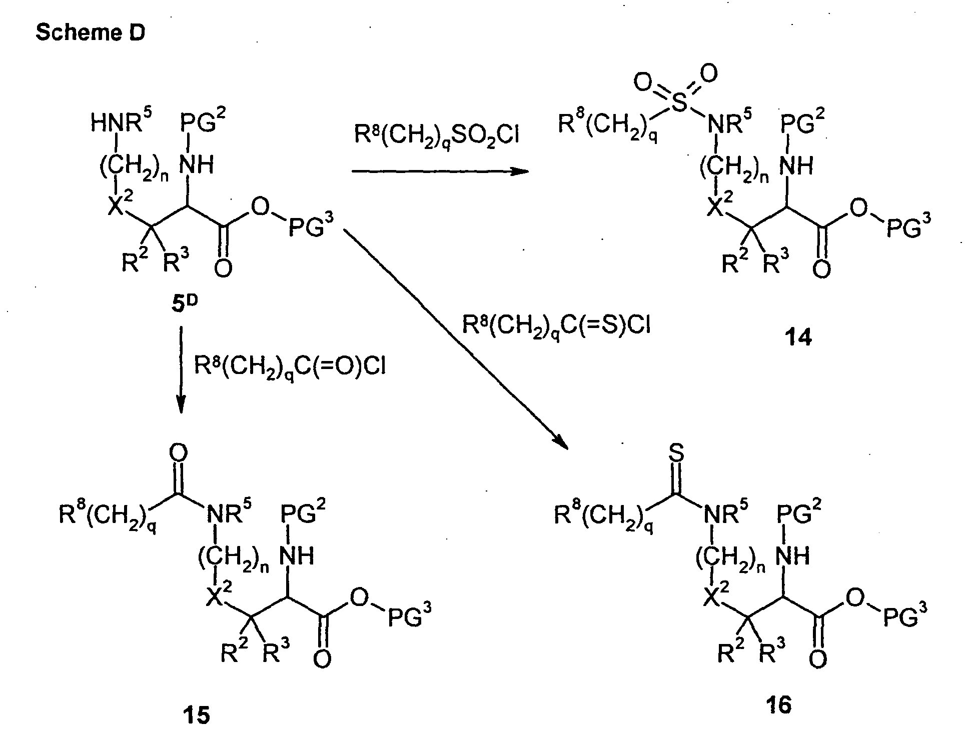

In Scheme D, compound **5**^{**D**} is the diamino acid derivative **5** where the ω-protecting group is a hydrogen atom. The free amine group reacts readily with sulphonyl chlorides, acyl chlorides and thioacyl chlorides, usually in the presence of a tertiary amine, to produce sulphonamides **14,** amides **15** and thioamides **16** respectively. The reagents are generally either available *per* se, or can be prepared from the corresponding acids. The reaction of scheme D is generally applicable to all the variations of the group R⁸(CH₂)_{q}, with the proviso that certain of the substituents contemplated for the phenyl and heteroaryl rings which are options for R⁸ may require protection. Such substituents and the appropriate protection will generally be obvious to those familiar with the art.

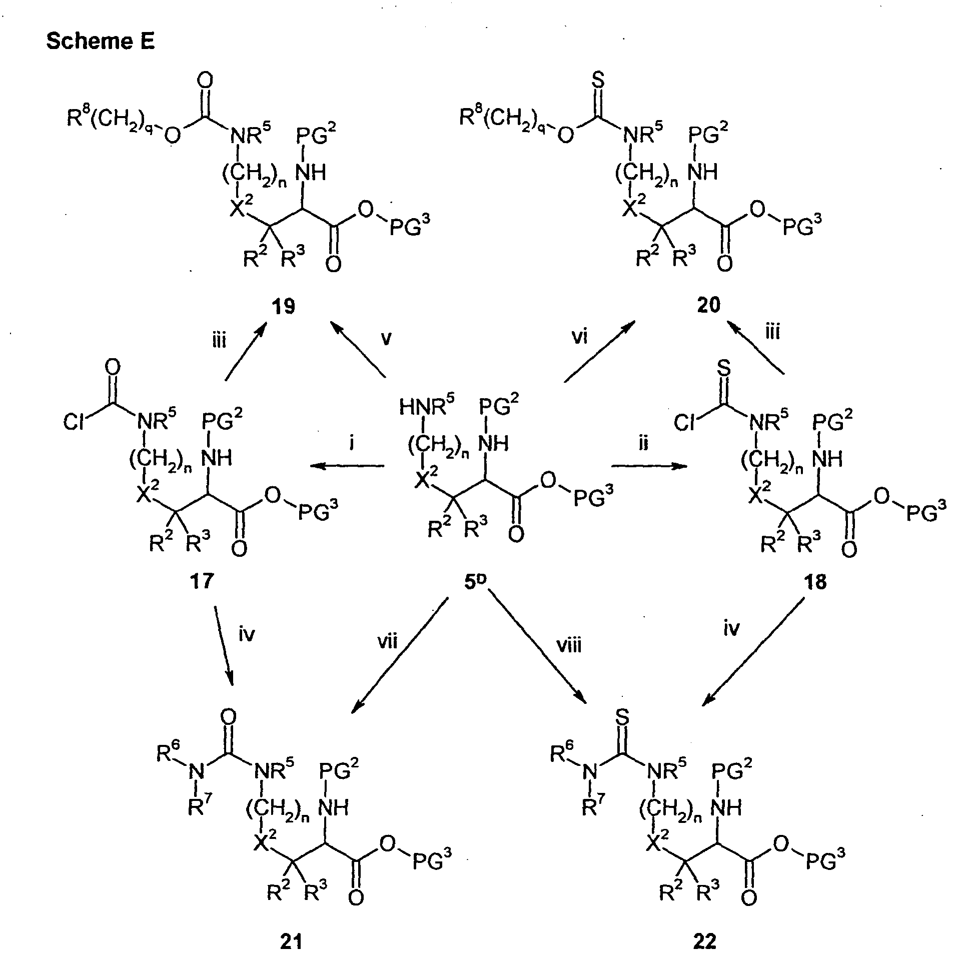

*Reagents:* i) COCl₂; ii) CSCl₂; iii) R⁸(CH₂)_{q}OH; iv) R⁶R⁷NH; v) R⁸(CH₂)_{q}OCOCl; vi) R⁸(CH₂)_{q}OCSCl; vii) R⁶R⁷NCOCl; viii) R⁶R⁷NCSCl.

Scheme E illustrates the elaboration of **5**^{**D**} to give carbamates and ureas, and their thio analogues. When R⁵ is other than a hydrogen atom, compound **5**^{**D**} can be converted to the corresponding carbamoyl chloride **17** or thiocarbamoyl chloride **18** by reaction with phosgene or thiophosgene. Other reagents are known in the art to be functionally equivalent to these toxic reagents and they may also be used. When R⁵ is hydrogen, the intermediate formed is an isocyanate or isothiocyanate, but this behaves functionally as an equivalent of the corresponding chloride. Intermediates **17** and **18** are not normally isolated, but are treated directly with alcohols to give carbamates **19** and thiocarbamates **20.** Treatment of these same intermediates with amines leads to the formation of ureas **21** and thioureas **22.** Alternatively, **5**^{**D**} may be reacted directly with a chloroformate or chlorothioformate to give the carbamate or thiocarbamate, or, when neither R⁶ nor R⁷ is hydrogen, with a chloroformamide or chlorothioformamide to give the urea or thiourea. When R⁶ or R⁷ is a hydrogen atom the chloroformamide or chlorothioformamide will tend to be unstable, in which case the isocyanate (e.g. R⁶-NCO) or isothiocyanate (e.g. R⁶-NCS) is used. As discussed previously for Scheme D, certain substituents within the embodiments of R⁸ may require appropriate protection.

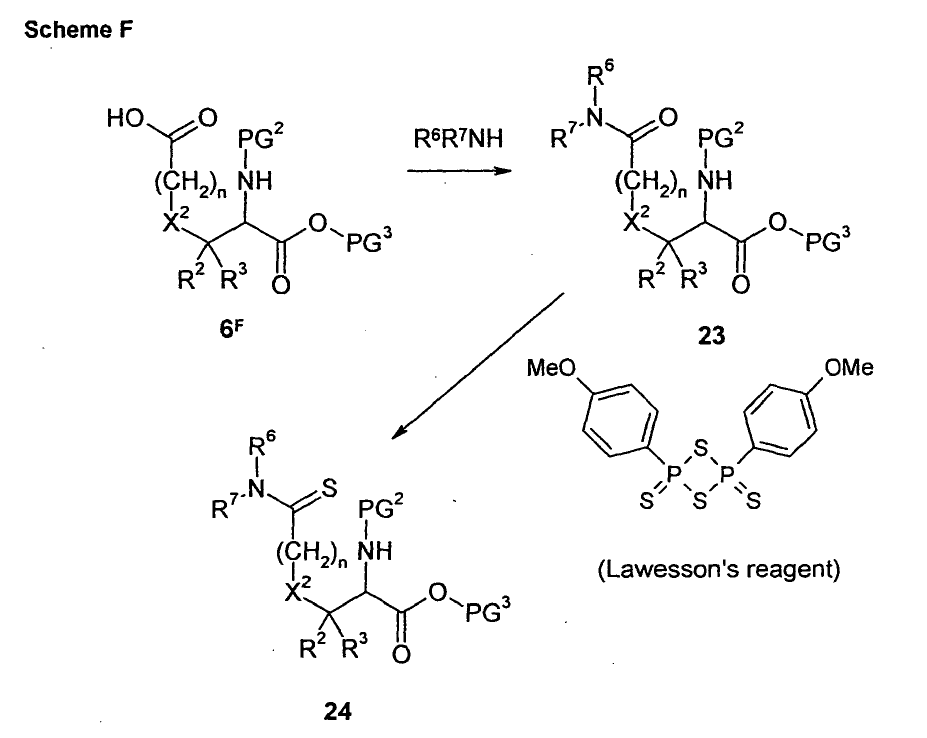

Scheme F illustrates the elaboration of the side chain of the amino dicarboxylic acid series. The ω-deprotected acid **6**^{**F**} can be reacted under a variety of conditions with an amine to give amide **23.** The condensation may be promoted by a dehydrating reagent such as a carbodiimide or a phosphorus reagent. Alternatively the acid may be converted into a more reactive derivative, such as by treatment with oxalyl chloride or thionyl chloride to give the corresponding acid chloride, which will react directly with an amine. The thioamide **24** may be obtained by treating the amide **23** with Lawesson's reagent.

When all the groups have been elaborated the final protecting group is removed and the product is isolated and purified using standard techniques.

These general methods are further illustrated in the following, non limiting examples.

### EXAMPLES

### Abbreviations

The following abbreviations have been used.
- DMF: N,N-Dimethyformamide
- h: Hour(s)
- hplc: High pressure liquid chromatography
- min: Minute(s)
- pet. ether: Petroleum ether fraction boiling at 60-80°C
- PyBOP®: (Benzotriazol-1-yloxy)tripyrrolidinophosphonium hexafluorophosphate
- PyBroP®: Bromotripyrrolidinophosphonium hexafluorophosphate
- TFA: Trifluoroacetic acid

### EXAMPLE 1

### (2S)-1-[N^{ω}-(Pyrazinyl-2-carbonyl)-L-ornithinyl]pyrrolidine-2-carbonitrile trifluoroacetate

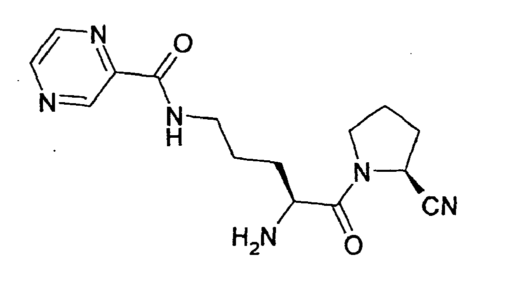

### A. N-(2-Nitrobenzenesulphenyl)-L-proline

L-Proline (25g, 217mmol) was dissolved in 2M NaOH (110mL, 220mmol) and dioxan (120mL). A solution of 2-nitrobenzenesulphenyl chloride (42g, 222mmol) in dioxan (60mL) was slowly added at the same time as 2M NaOH (110mL, 220mmol). After 2h at room temperature the reaction mixture was poured into water (500mL) and the solid filtered off. The pH of the filtrate was adjusted to pH3 with 2M HCl and the solution was extracted with ethyl acetate (3 x 500mL). The combined organic extracts were washed with water (4 x 200mL) and brine (1 x 200mL), dried (Na₂SO₄) and evaporated *in vacuo* to give an orange solid identified as *N*-(2-nitrobenzenesulphenyl)-L-proline (58.1g, 217mmol, 100%).

### B. N-(2-Nitrobenzenesulphenyl)-L-proline succinimidyl ester

*N*-(2-Nitrobenzenesulphenyl)-L-proline (57.9g, 216mmol) was dissolved in CH₂Cl₂/DMF (9:1, 500mL). N-Hydroxysuccinimide (37.3g, 324mmol) and water-soluble carbodiimide (51.8g, 260mmol) were added. After 18h at room temperature the solvent was removed *in vacuo* and the residue was taken up in ethyl acetate (1000mL). The solution was washed with water (4 x 200mL) and brine (1 x 200mL), dried (Na₂SO₄) and evaporated *in vacuo* to give a yellow solid identified as *N*-(2-nitrobenzenesulphenyl)-L-proline succinimidyl ester (78.9g, 216mmol, 100%).

### C. N-(2-Nitrobenzenesulphenyl)-L-prolinamide

N-(2-Nitrobenzenesulphenyl)-L-proline succinimidyl ester (78.5g, 215mmol) was dissolved in dioxan (500mL). Ammonia (35%, 100mL) was added. After stirring at room temperature for 2h the reaction mixture was poured into water (700mL). The precipitate was filtered off, washed with water (200mL), dried over P₂O₅ and recrystallised from ethyl acetate/pet ether to give a yellow solid identified as N-(2-nitrobenzenesulphenyl)-L-prolinamide (49.6g, 185mmol, 86%).

### D. (2S)-N-(2-Nitrobenzenesulphenyl)pyrrolidine-2-carbonitrile

*N*-(2-Nitrobenzenesulphenyl)-L-prolinamide (49g, 183mmol) was dissolved in dry THF(300mL). The solution was cooled to 0°C, triethylamine (36.7g, 367mmol) was added followed by the slow addition of trifluoroacetic anhydride (77g, 367mmol). The pH was adjusted to pH9 with triethylamine. After 30min the reaction mixture was diluted with ethyl acetate (500mL), washed with water (1 x 200mL) and brine (1 x 200mL), dried (Na₂SO₄) and evaporated *in vacuo* to give an orange oil which was purified by flash chromatography (eluant: 80% pet ether, 20% ethyl acetate) to give a yellow solid identified as (2*S*)-*N*-(2-nitrobenzenesulphenyl)pyrrolidine-2-carbonitrile (38.9g, 150mmol, 82%).

### E. (2S)-Pyrrolidine-2-carbonitrile hydrochloride

(2*S*)-*N*-(2-Nitrobenzenesulphenyl)pyrrolidine-2-carbonitrile (38.5g, 149mmol) was dissolved in diethyl ether (200mL). 4M HCl/Dioxan (150mL, 600mmol) was slowly added. After 2h at room temperature the reaction mixture was poured into diethyl ether (1000mL). The solid was filtered off, washed with diethyl ether (500mL) and recrystallised from methanol/diethyl ether to give a white solid identified as (2*S*)-pyrrolidine-2-carbonitrile hydrochloride (18.9g, 142.5mmol, 96%).

### F. (2S)-1-[N^{α}-(tert-Butyloxycarbonyl)-N^{ω}-(pyrazinyl-2-carbonyl)-L-ornithinyl]-pyrrolidine-2-carbonitrile.

*N*^{α}-(*tert*-Butyloxycarbonyl)-*N*^{ω}-(pyrazinyl-2-carbonyl)-L-ornithine (2.5g, 7.4mmol) was dissolved in CH₂Cl₂ (50mL). This solution was cooled to 0°C, (2*S*)-pyrrolidine-2-carbonitrile hydrochloride (1.2g, 9.1 mmol) and PyBOP® (4.3g, 8.23mmol) were added, and the pH adjusted to pH9 with triethylamine. After 18h at 0°C to room temperature the solvent was removed *in vacuo* and the residue was taken up in ethyl acetate (200mL). This solution was washed with 0.3M KHSO₄ (2 x 50mL), sat. NaHCO₃ (2 x 50mL), water (2 x 50mL) and brine (1 x 50mL), dried (Na₂SO₄) and evaporated *in vacuo* to give a yellow oil. This was purified by flash chromatography (eluant: 80% ethyl acetate, 20% pet. ether) to give a colourless oil identified as (2*S*)-1-[*N*^{α}-(*tert*-butyloxycarbonyl)-*N*^{ω}-(pyrazinyl-2-carbonyl)-L-ornithinyl]pyrrolidine-2-carbonitrile (2.98g, 7.16mmol, 97%).

### G. (2S)-1-[N^{ω}-(pyrazinyl-2-carbonyl)-L-ornithinyl]pyrrolidine-2-carbonitrile trifluoroacetate

(2*S*)-1-[*N*^{α}-*tert*-Butyloxycarbonyl-*N*^{ω}-(pyrazinyl-2-carbonyl)-L-ornithinyl]pyrrolidine-2-carbonitrile (2.8g, 6.7mmol) was dissolved in trifluoroacetic acid (5mL). After 1h at room temperature the solvent was removed *in vacuo.* The residue was purified by preparative hplc (Vydac C18, 5 to 50% 0.1% TFA/acetonitrile into 0.1% TFA/water over 40min at 3mUmin). Fractions containing the product were lyophilised to give a colourless oil identified as (2*S*)-1-[*N*^{ω}-(pyrazinyl-2-carbonyl)-L-ornithinyl]pyrrolidine-2-carbonitrile trifluoroacetate (1.5g, 3.48mmol, 52%).
[M+H]⁺ = 317.3

### EXAMPLE 2

### (2S)-1-[N^{ω}-(Pyrazinyl-2-carbonyl)-L-lysinyl]pyrrolidine-2-carbonitrile trifluoroacetate

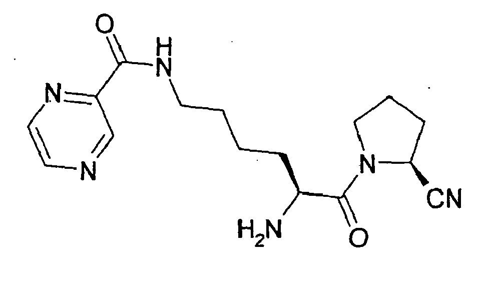

### A. (N^{α}-(tert-Butyloxycarbonyl)-N^{ω}-(9-fluorenylmethyloxycarbonyl)-L-lysinyl)-L-prolinamide

*N*^{α}-(*tert*-Butyloxycarbonyl)-*N*^{ω}-(9-fluorenylmethyloxycarbonyl)-L-lysine (5g, 10.7mmol) was dissolved in CH₂Cl₂ (100mL). The solution was cooled to 0°C, L-prolinamide (1.78g, 11.7mmol) and PyBOP® (6.7g, 12.8mmol) were added, and the pH adjusted to pH9 with triethylamine. After 18h at 0°C to room temperature the solvent was removed *in vacuo* and the residue was taken up in ethyl acetate (200mL). The solution was washed with 0.3M KHSO₄ (2 x 50mL), sat. NaHCO₃ (2 x 50mL), water (2 x 50mL) and brine (1 x 50mL), dried (Na₂SO₄) and evaporated *in vacuo.* The residue was purified by flash chromatography (eluant: 2% methanol, 98% chloroform) to give a colourless oil identified as (*N*^{α}-(*tert*-butyloxycarbonyl)-*N*^{ω}-(9-fluorenylmethyloxycarbonyl)-L-lysinyl)-L-prolinamide (4.05g, 7.2mmol, 67%).

### B. (2S)-1-(N^{α}-(tert-Butyloxycarbonyl)-N^{ω}-(9-fluorenylmethyloxycarbonyl)-L-lysinyl)-pyrrolidine-2-carbonitrile

(*N*^{α}-(*tert*-Butyloxycarbonyl)-*N*^{ω}-(9-fluorenylmethyloxycarbonyl)-L-lysinyl)-L-prolinamide (3.95g, 7.02mmol) was dissolved in dry THF (100mL). The solution was cooled to 0°C, triethylamine (1.4g, 14mmol) was added followed by the slow addition of trifluoroacetic anhydride (2.97g, 14.1mmol). The pH was adjusted to pH9 with triethylamine. After 30min the reaction mixture was diluted with ethyl acetate (100mL), washed with water (1 x 50mL) and brine (1 x 50mL), dried (Na₂SO₄) and evaporated *in vacuo* to give an orange oil. The residue was purified by flash chromatography (eluant: 60% pet ether, 40% ethyl acetate) to give a colourless oil identified as (2*S*)-1-(*N*^{α}-(*tert*-butyloxycarbonyl)-*N*^{ω}-(9-fluorenylmethyloxycarbonyl)-L-lysinyl)pyrrolidine-2-carbonitrile (3.3g, 6.11mmol, 87%).

### C. (2S)-1-(N^{α}-(tert-Butyloxycarbonyl)-L-lysinyl)pyrrolidine-2-carbonitrile

(2*S*)-1-(*N*^{α}-(*tert*-Butyloxycarbonyl)-*N*^{ω}-(9-fluorenylmethyloxycarbonyl)-L-lysinyl)pyrrolidine-2-carbonitrile (3.1g, 5.7mmol) was dissolved in THF (80mL). Diethylamine (20mL) was added. After 2h at room temperature the solvent was removed *in vacuo.* The residue was purified by flash chromatography (eluant: 90% chloroform, 7% methanol, 3% triethylamine) to give a colourless oil identified as (2*S*)-1-(*N*^{α}-(*tert*-butytoxycarbonyl)-L-lysinyl)pyrrolidine-2-carbonitrile (1.63g, 5.03mmol, 89%).

### D. (2S)-1-(N^{α}-(tert-Butyloxycarbonyl)-N^{ω}-(pyrazinyl-2-carbonyl)-L-lysinyl)pyrrolidine-2-carbonitrile

(2*S*)-1-(*N*^{α}-(*tert*-Butyloxycarbonyl)-L-lysinyl)pyrrolidine-2-carbonitrile (100mg, 0.31mmol) was dissolved in CH₂Cl₂/DMF (9:1, 20mL). To this solution at 0°C was added 1-hydroxybenzotriazole hydrate (84mg, 0.62mmol), water-soluble carbodiimide (76mg, 0.38mmol), 2-pyrazinecarboxylic acid (43mg, 0.35mmol) and triethylamine (65mg, 0.65mmol). After 18 h at 0°C to room temperature the solvent was removed *in vacuo* and the residue was taken up in ethyl acetate (70mL). This solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo* to give a yellow oil. The residue was purified by flash chromatography (eluant: 2% methanol, 98% chloroform) to give a colourless oil identified as (2*S*)-1-(*N*^{α}-(*tert*-butyloxycarbonyl)-*N*^{ω}-(pyrazinyl-2-carbonyl)-L-lysinyl)pyrrolidine-2-carbonitrile (124mg, 0.29mmol, 93%).

### E. (2S)-1-[N^{ω}-(Pyrazinyl-2-carbonyl)-L-lysinyl]pyrrolidine-2-carbonitrile trifluoroacetate

(2*S*)-1-(*N*^{α}-(*tert*-Butyloxycarbonyl)-*N*^{ω}-(pyrazinyl-2-carbonyl)-L-lysinyl)pyrrolidine-2-carbonitrile (110mg, 0.26mmol) was dissolved in trifluoroacetic acid (5mL). After 1 h at room temperature the solvent was removed *in vacuo*. The residue was purified by preparative hplc (Vydac C18, 5 to 50% 0.1% TFA/acetonitrile into 0.1% TFA/water over 40min at 3mUmin). Fractions containing the product were lyophilised to give a colourless oil identified as (2*S*)-1-[*N*^{ω}-(pyrazinyl-2-carbonyl)-L-lysinyl]pyrrolidine-2-carbonitrile trifluoroacetate (66mg).
[M+H]⁺ = 331.1

### EXAMPLE 3

### (4R)-3-[N^{ω}-(Pyrazinyl-2-carbonyl)-L-lysinyl]thiazolidine-4-carbonitrile trifluoroacetate

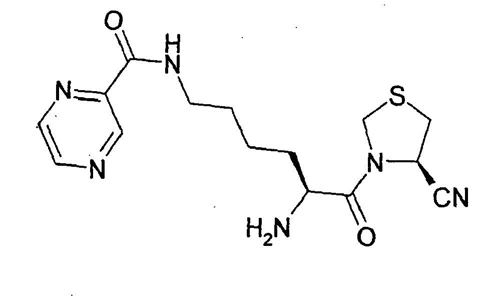

### A. (4R)-3-(tert-Butyloxycarbonyl)thiazolidine-4-carboxamide

(4*R*)-3-(*tert*-Butyloxycarbonyl)thiazolidine-4-carboxylic acid (12.5g,54.1mmol) was dissolved in CH₂Cl₂ /DMF (9:1, 150mL). To this solution at 0°C was added 1-hydroxybenzotriazole hydrate (14.6g, 108mmol) and water-soluble carbodiimide (13.0g, 65mmol). After 1 h at 0°C ammonia (35%, 50mL) was added. After 18h at 0°C to room temperature the solvent was removed *in vacuo* and the residue was taken up in ethyl acetate (500mL). The solution was washed with 0.3M KHSO₄ (2 x 100mL), sat. NaHCO₃ (2 x 100mL), water (2 x 100mL) and brine (1 x 100mL), dried (Na₂SO₄) and evaporated *in vacuo* to give a yellow oil. The residue was purified by flash chromatography (eluant: 2% methanol, 98% chloroform) to give a colourless oil identified as (4*R*)-3-(*tert-*butyloxycarbonyl)thiazolidine-4-carboxamide (8.9g, 38.4mmol, 71%).

### B. (4R)-Thiazolidine-4-carboxamide hydrochloride

(4*S*)-3-(*tert*-Butyloxycarbonyl)thiazolidine-4-carboxamide (8.6g, 37.1mmol) was dissolved in 4M HCl/dioxan (50mL). After 1 h at room temperature the solvent was evaporated in *vacuo* to give a white solid identified as (4R)-thiazolidine-4-carboxamide hydrochloride (6.2g, 36.8mmol, 99%).

### C. (4R)-3-[N^{α}-(tert-Butyloxycarbonyl)-N^{ω}-(9-fluorenylmethyloxycarbonyl)-L-lysinyl]-thiazolidine-4-carboxamide

*N*^{α}-(*tert*-Butyloxycarbonyl)-*N*^{ω}-(9-fluorenylmethyloxycarbonyl)-L-lysine (5g, 10.7mmol) was dissolved in CH₂Cl₂ (100mL). This solution was cooled to 0°C, (4*R*)-thiazolidine-4-carboxamide hydrochloride (1.78g, 11.7mmol) and PyBOP® (6.7g, 12.8mmol) were added, and the pH was adjusted to pH9 with triethylamine. After 18h at 0°C to room temperature the solvent was removed *in vacuo* and the residue was taken up in ethyl acetate (200mL). The solution was washed with 0.3M KHSO₄ (2 x 50mL), sat. NaHCO₃ (2 x 50mL), water (2 x 50mL) and brine (1 x 50mL), dried (Na₂SO₄) and evaporated *in vacuo* to give a yellow oil. The residue was purified by flash chromatography (eluant: 2% methanol, 98% chloroform) to give a colourless oil identified as (4*R*)-3-[*N*^{α}-(*tert*-butyloxycarbonyl)-*N*^{ω}-(9-fluorenylmethyloxycarbonyl)-L-lysinyl]thiazolidine-4-carboxamide (2.81 g, 4.8mmol, 44%).

### D. (4R)-3-[N^{α}-(tert-Butyloxycarbonyl)-N^{ω}-(9-fluorenylmethyloxycarbonyl)-L-lysinyl]-thiazolidine-4-carbonitrile

(4*R*)-3-[*N*^{α}-(*tert*-Butyloxycarbonyl)-*N*^{ω}-(9-fluorenylmethyloxycarbonyl)-L-lysinyl]thiazolidine-4-carboxamide (2.7g, 4.7mmol) was dissolved in dry THF (100mL). The solution was cooled to 0°C, triethylamine (1.0g, 10mmol) was added followed by the slow addition of trifluoroacetic anhydride (2.0g, 9.5mmol). The pH was adjusted to pH9 with triethylamine. After 30min the reaction mixture was diluted with ethyl acetate (100mL), washed with water (1 x 50mL) and brine (1 x 50mL), dried (Na₂SO₄) and evaporated *in vacuo*. The residue was purified by flash chromatography (eluant: 60% pet ether, 40% ethyl acetate) to give a colourless oil identified as (4*R*)-3-[*N*^{α}-(*tert*-butyloxycarbonyl)-*N*^{ω}-(9-fluorenylmethyloxycarbonyl)-L-lysinyl]thiazolidine-4-carbonitrile (2.14g, 3.81 mmol, 82%).

### E. (4R)-3-[N^{α}-(tert-Butyloxycarbonyl)-L-lysinyl]thiazolidine-4-carbonitrile

(4*R*)-3-[*N*^{α}-(*tert*-Butyloxycarbonyl)-*N*^{ω}-(9-fluorenylmethyloxycarbonyl)-L-lysinyl]thiazolidine-4-carbonitrile (1.9g, 3.4mmol) was dissolved in THF (40mL). Diethylamine (10mL) was added. After 2h at room temperature the solvent was removed *in vacuo.* The residue was purified by flash chromatography (eluant: 90% chloroform, 7% methanol, 3% triethylamine) to give a colourless oil identified as (4*R*)-3-[*N*^{α}-(*tert*-butyloxycarbonyl)-L-lysinyl]thiazolidine-4-carbonitrile (863mg, 2.5mmol, 75%).

### F. (4R)-3-[N^{α}-(tert-Butyloxycarbonyl)-N^{ω}-(pyrazinyl-2-carbonyl)-L-lysinyl]thiazolidine-4-carbonrtrile

(4*R*)-3-[*N*^{*α*}-(*tert*-Butyloxycarbonyl)-L-lysinyl]thiazolidine-4-carbonitrile (100mg, 0.29mmol) was dissolved in CH₂Cl₂ (20mL). To this solution at 0°C 2-pyrazinecarboxylic acid (43mg, 0.35mmol) and PyBOP® (170mg, 0.33mmol) were added and the pH was adjusted to pH9 with triethylamine. After 18 h at 0°C to room temperature the solvent was removed *in* vacuo and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo*. The residue was purified by flash chromatography (eluant: 2% methanol, 98% chloroform) to give a colourless oil identified as (4*R*)-3-[*N*^{α}-(*tert*-butyloxycarbonyl)-*N*^{ω}-(pyrazinyl-2-carbonyl)-L-lysinyl]thiazolidine-4-carbonitrile (112mg, 0.25mmol, 86%).

### G. (4R)-3-[N^{ω}-(Pyrazinyl-2-carbonyl)-L-lysinyl]thiazolidine-4-carbonitrile trifluoroacetate

(4*R*)-3-[*N*^{α}-(*tert*-Butyloxycarbonyl)-*N*^{ω}-(pyrazinyl-2-carbonyl)-L-lysinyl]thiazolidine-4-carbonitrile (110mg, 0.26mmol) was dissolved in trifluoroacetic acid (5mL). After 1h at room temperature the solvent was removed *in vacuo*. The residue was purified by preparative hplc (Vydac C18, 5 to 50% 0.1% TFA/acetonitrile into 0.1% TFA/water over 40min at 3mUmin). Fractions containing the product were lyophilised to give a colourless oil identified as (4*R*)-3-[*N*^{ω}-(pyrazinyl-2-carbonyl)-L-lysinyl]thiazolidine-4-carbonitrile trifluoroacetate (57mg).
[M+H]⁺ = 349.1

### EXAMPLE 4

### (2S)-1-[N^{ω}-(Pyridyl-3-methyl)-L-glutaminyl]pyrrolidine-2-carbonitrile dihydrochloride

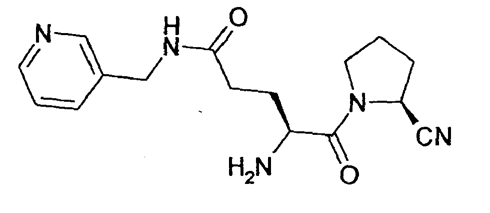

### A. (2S)-1-[N-(tert-Butyloxycarbonyl)-O^{ω}-methyl-L-glutamyl]pyrrolidine-2-carbonitrile

*N*-(*tert*-Butyloxycarbonyl)-*O*^{ω}-methyl-L-glutamic acid (1.0g, 3.83mmol) was dissolved in CH₂Cl₂ /DMF (9:1, 20mL). To this solution at 0°C were added 1-hydroxybenzotriazole hydrate (788mg, 5.84mmol), water-soluble carbodiimide (877mg, 4.38mmol), (2S)-pyrrolidine-2-carbonitrile hydrochloride (609mg, 4.6mmol) and triethylamine (65mg, 0.65mmol). After 18h at 0°C to room temperature the solvent was removed *in vacuo* and the residue was taken up in ethyl acetate (70mL), this solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo.* The residue was purified by flash chromatography (eluant: 50% ethyl acetate, 50% pet. ether) to give a brown oil identified as (2*S*)-1-[*N*-(*tert-*butyloxycarbonyl)-*O*^{ω}-methyl-L-glutamyl]pyrrolidine-2-carbonitrile (290mg, 0.86mmol, 22%).

### B. (2S)-1-[N-(tert-Butyloxycarbonyl)-L-glutamyl]pyrrolidine-2-carbonitrile

(2*S*)-1-[*N*-(*tert*-Butyloxycarbonyl)-*O*^{ω}-methyl-L-glutamyl]pyrrolidine-2-carbonitrile (250mg, 0.74mmol) was dissolved in dioxan (5mL), 1M Lithium hydroxide (1.1mL, 1.1mmol) was added. After 1 h at room temperature the solvent was removed *in vacuo* and the residue was taken up in ethyl acetate (70mL). This solution was washed with 1 M KHSO₄ (2 x 20mL), water (2 x 20mL) and brine (1 x 20mL), dried (Na₂SO₄) and evaporated *in vacuo* to give a colourless oil identified as (2*S*)-1-[*N*-(*tert*-butyloxycarbonyl)-L-glutamyl]pyrrolidine-2-carbonitrile (200mg, 0.61mmol, 83%).

### C. (2S)-1-[N^{α}-(tert-Butyloxycarbonyl)-N^{ω}-(pyridyl-3-methyl)-L-glutaminyl]pyrrolidine-2-carbonitrile

(2*S*)-1-[*N*-(*tert*-Butyloxycarbonyl)-L-glutamyl]pyrrolidine-2-carbonitrile (30mg, 0.093mmol) was dissolved in CH₂Cl₂/DMF (9:1, 10mL). To this solution at 0°C was added 1-hydroxybenzotriazole hydrate (21mg, 0.16mmol), water-soluble carbodiimide (21mg, 0.105mmol), 3-(aminomethyl)pyridine (11mg, 0.1mmol) and triethylamine(20mg, 0.2mmol). After 18h at 0°C to room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo* to give a yellow oil. The residue was purified by flash chromatography (eluant: 5% methano, 97% chloroform) to give a colourless oil identified as (2*S*)-1-[*N*^{α}-(*tert-*butyloxycarbonyl)-*N*^{ω}-(pyridyl-3-methyl)-L-glutaminyl]pyrrolidine-2-carbonitrile (16.6mg, 0.04mmol, 44%).

### D. (2S)-1-[N^{ω}-(Pyridyl-3-methyl)-L-glutaminyl]pyrrolidine-2-carbonitrile dihydrochloride

(2*S*)-1-[*N*^{α}-(*tert*-Butyloxycarbonyl)-*N*^{ω}-(pyridyl-3-methyl)-L-glutaminyl]pyrrolidine-2-carbonitrile (17mg, 0.04mmol) was dissolved in 4M HCl/dioxan (5mL). After 1h at room temperature the solvent was removed *in vacuo* to give a white solid identified as (2S)-1-[*N*^{ω}-(pyridyl-3-methyl)-L-glutaminyl]pyrrolidine-2-carbonitrile dihydrochloride (17mg, 0.04mmol, 100%).
[M+H]⁺ = 316.2

### EXAMPLE 5

### 1-[N^{ω}-(Pyrazinyl-2-carbonyl)-L-ornithinyl]pyrrolidine trifluoroacetate

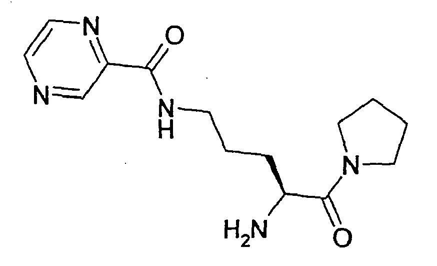

### A. 1-[N^{ω}-(Benzyloxycarbonyl)-N^{α}-(tert-butyloxycarbonyl)-L-ornithinyl]pyrrolidine

*N*^{ω}-(Benzyloxycarbonyl)-*N*^{α}-(*tert*-butyloxycarbonyl)-L-ornithine (5.49g, 15mmol) was dissolved in CH₂Cl₂ /DMF (9:1, 100mL). To this solution at 0°C was added 1-hydroxybenzotriazole hydrate (3.37g, 22mmol), water-soluble carbodiimide (3.46g, 18mmol), pyrrolidine (1.28g, 18mmol) and triethylamine (200mg, 20mmol). After 18h at 0°C to room temperature the solvent was removed *in vacuo* and the residue was taken up in ethyl acetate (200mL). The solution was washed with 0.3M KHSO₄ (2 x 50mL), sat. NaHCO₃ (2 x 50mL), water (2 x 50mL) and brine (1 x 50mL), dried (Na₂SO₄) and evaporated *in vacuo*. The residue was purified by flash chromatography (eluant: 90% ethyl acetate, 10% pet. ether) to give a colourless oil identified as 1-[*N*^{ω-}(benzyloxycarbonyl)-*N*^{α}-(*tert*-butyloxycarbonyl)-L-ornithinyl]pyrrolidine (5.15g, 12.3mmol, 82%).

### B. 1-[N^{α}-(tert-Butyloxycarbonyl)-L-ornithinyl]pyrrolidine

1-[*N*^{ω}-(Benzyloxycarbonyl)-*N*^{α}-(*tert*-butyloxycarbonyl)-L-ornithinyl]pyrrolidine (2.15g, 5.13mmol) was dissolved in methanol (80mL). This solution was hydrogenated over 10% Pd/C (400mg). After 2h the catalyst was filtered off and washed with methanol (50mL). The combined filtrates were evaporated *in vacuo* to give an off white solid identified as 1-[*N*^{α}-(*tert*-butyloxycarbonyl)-L-ornithinyl]pyrrolidine (1.35g, 4.74mmol, 94%).

### C. 1-[N^{α}-(tert-Butytoxycarbonyl)-N^{α}-(pyrazinyl-2-carbonyl)-L-ornithinyl]pyrrotidine

1-[*N*^{α}-(*tert*-Butyloxycarbonyl)-L-omithinyl]pyrrolidine (100mg, 0.35mmol) was dissolved in CH₂Cl₂ (20mL). To this solution at 0°C were added PyBroP® (195mg, 0.4mmol), 2-pyrazinecarboxylic acid (50mg, 0.4mmol) and triethylamine (100mg, 1.0mmol). After 18h at 0°C to room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo*. The residue was purified by flash chromatography (eluant: 3% methanol, 97% chloroform) to give a sticky white solid identified as 1-[*N*^{α}-(*tert-*butyloxycarbonyl)-*N*^{ω}-(pyrazinyl-2-carbonyl)-L-ornithinyl]pyrrolidine (90mg, 0.25mmol, 66%).

### D. 1-[N^{ω}-(Pyrazinyl-2-carbonyl)-L-ornithinyl]pyrrolidine trifluoroacetate

1-[*N*^{α}-(*tert*-Butyloxycarbonyl)-*N*^{ω}-(pyrazinyl-2-carbonyl)-L-ornithinyl]pyrrolidine (90mg, 0.23mmol) was dissolved in 4M HCl/dioxan (15mL). After 45min at room temperature the solvent was removed *in vacuo*. The residue was purified by preparative hplc (Vydac C18, 5 to 50% 0.1% TFA/acetonitrile into 0.1% TFA/water over 40min at 3mUmin). Fractions containing the product were lyophilised to give a colourless oil identified as 1-[*N*^{ω-}(pyrazinyl-2-carbonyl)-L-ornithinyl]pyrrolidine trifluoroacetate (51mg).
[M+H]⁺ = 292.1

### EXAMPLE 6

### 3-[N^{ω}-(Pyrazinyl-2-carbonyl)-L-ornithinyl]thiazolidine trifluoroacetate

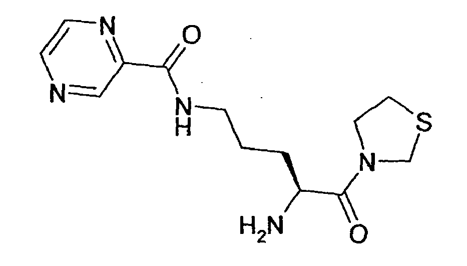

### A. 3-[N^{α}-(tert-Butyloxycarbonyl)-N^{ω}-(9-fluorenylmethyloxycarbonyl)-L-ornithinyl]thiazolidine

*N*^{α}-(*tert*-Butyloxycarbonyl)-*N*^{ω}-(9-fluorenylmethyloxycarbonyl)-L-ornithine (2.73g, 6mmol) was dissolved in CH₂Cl₂ /DMF (9:1, 100mL). To this solution at 0°C were added 1-hydroxybenzotriazole hydrate (1.53g, 10mmol), water-soluble carbodiimide (1.34g, 7mmol), thiazolidine (1.28g, 18mmol) and triethylamine (80mg, 8mmol). After 18h at 0°C to room temperature the solvent was removed *in vacuo* and the residue was taken up in ethyl acetate (100mL). The solution was washed with 0.3M KHSO₄ (2 x 25mL), sat. NaHCO₃ (2 x 25mL), water (2 x 25mL) and brine (1 x 25mL), dried (Na₂SO₄) and evaporated *in vacuo*. The residue was purified by flash chromatography (eluant: 75% ethyl acetate, 25% pet. ether) to give a white solid identified as 3-[*N*^{α}-(*tert-*butyloxycarbonyl)-*N*^{ω}-(9-fluorenylmethyloxycarbonyl)-L-ornithinyl]thiazolidine (2.55g, 4.85mmol, 81%).

### B. 3-[N^{α}-(tert-Butyloxycarbonyl)-L-ornithinyl]thiazolidine

3-[*N*^{α}-(*tert*-Butyloxycarbonyl)-*N*^{ω}-(9-fluorenylmethyloxycarbonyl)-L-ornithinyl]thiazolidine (1.15g, 2.13mmol) was dissolved in acetonitrile (20mL). Diethylamine (5mL) was added. After 90min at room temperature the solvent was removed *in vacuo* and the residue was purified by flash chromatography (eluant: 90% chloroform, 7% methanol, 3% triethylamine) to give a pale yellow oil identified as 3-[*N*^{*α*}-(*tert-*butyloxycarbonyl)-L-ornithinyl]thiazolidine (530mg, 1.67mmol, 78%).

### C. 3-[N^{α}-(tert-Butyloxycarbonyl)-N^{ω}-(pyrazinyl-2-carbonyl)-L-ornithinyl]thiazolidine

3-[*N*^{α}-(*tert*-Butyloxycarbonyl)-L-omithinyl]thiazolidine (80mg, 0.27mmol) was dissolved in CH₂Cl₂ (20mL). To this solution at 0°C were added PyBroP® (146mg, 0.3mmol), 2-pyrazinecarboxylic acid (37mg, 0.3mmol) and triethylamine (90mg, 0.9mmol). After 18h at 0°C to room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo.* The residue was purified by flash chromatography (eluant: 3% methanol, 97% chloroform) to give a sticky white solid identified as 3-[*N*^{*α*}-(*tert-*butyloxycarbonyl)-*N*^{ω}-(pyrazinyl-2-carbonyl)-L-ornithinyl]thiazolidine (45mg, 0.11mmol, 41%).

### D. 3-[N^{ω}-(Pyrazinyl-2-carbonyl)-L-ornithinyl]thiazolidine trifluoroacetate

3-[*N*^{α}-(*tert*-Butyloxycarbonyl)-*N*^{ω}-(pyrazinyl-2-carbonyl)-L-omithinyl]thiazolidine (45mg, 0.11mmol) was dissolved in 4M HCl/dioxan (10mL). After 45min at room temperature the solvent was removed *in vacuo*. The residue was purified by preparative hplc (Vydac C18, 5 to 50% 0.1% TFA/acetonitrile into 0.1% TFA/water over 40min at 3mL/min). Fractions containing the product were lyophilised to give a colourless oil identified as 3-[N^{ω-}(pyrazinyl-2-carbonyl)-L-ornithinyl]thiazolidine trifluoroacetate (14mg).
[M+H]⁺ = 310.0

### EXAMPLE 7

### (2S)-1-[S-(Acetylaminomethyl)-L-cysteinyl]pyrrolidine-2-carbonitrile trifluoroacetate

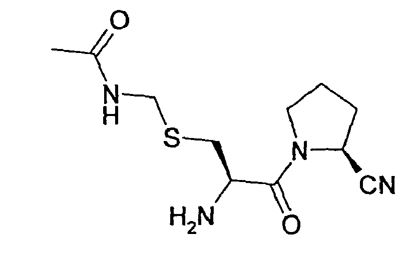

### A. (2S)-1-[S-(Acetylaminomethyl)-N-(tert-butyloxycarbonyl)-L-cysteinyl]pyrrolidine-2-carbonitrile

*S*-(Acetylaminomethyl)-*N*-(*tert*-butyloxycarbonyl)-L-cysteine (660mg, 2.26mmol) was dissolved in CH₂Cl₂ (30mL). To this solution at 0°C were added (2*S*)-pyrrolidine-2-carbonitrile hydrochloride (250mg, 1.89mmol) and PyBOP® (1.3g, 2.49mmol), and the pH adjusted to pH9 with triethylamine. After 18h at 0°C to room temperature the solvent was removed *in vacuo* and the residue was taken up in ethyl acetate (150mL). The solution was washed with 0.3M KHSO₄ (2 x 30mL), sat. NaHCO₃ (2 x 30mL), water (2 x 30mL) and brine (1 x 30mL), dried (Na₂SO₄) and evaporated *in vacuo*. The residue was purified by flash chromatography (eluant: 75% ethyl acetate, 25% pet. ether) to give a colourless oil identified as (2*S*)-1-[*S*-(acetylaminomethyl)-*N*-(*tert*-butyloxycarbonyl)-L-cysteinyl]-pyrrolidine-2-carbonitrile (650mg, 1.76mmol, 78%).

### B. (2S)-1-[S-(Acetylaminomethyl)-L-cysteinyl]pyrrolidine-2-carbonitrile trifluoroacetate

(2*S*)-1-[*S*-(Acetylaminomethyl)-*N*-(*tert*-butyloxycarbonyl)-L-cysteinyl]pyrrolidine-2-carbonitrile (610mg, 1.65mmol) was dissolved in trifluoroacetic acid (30mL). After 1h at room temperature the solvent was removed *in vacuo* to give a colourless oil identified as (2*S*)-1-[*S*-(acetylaminomethyl)-L-cysteinyl]pyrrolidine-2-carbonitrile trifluoroacetate (620mg, 1.61mmol, 98%).
[M+H]⁺ = 271.0

### EXAMPLE 8

### (2S)-1-[(2'R)-3'-(Acetylaminomethylthio)-2'-amino-3'-methylbutanoyl]pyrrolidine-2-carbonitrile trifluoroacetate

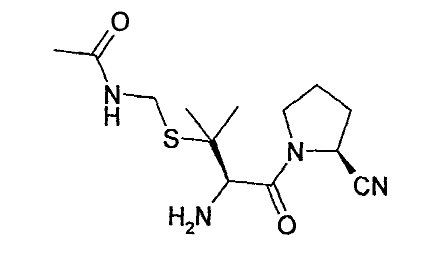

### A. (2S)-1-[(2'R)-3'-(Acetylaminomethylthio)-2'-(tert-butyloxycarbonylamino)-3'-methylbutanoyl]pyrrolidine-2-carbonitrile

*S*-(Acetylaminomethyl)-*N*-(*tert*-butyloxycarbonyl)penicillamine (720mg, 2.25mmol) was dissolved in CH₂Cl₂ (30mL). To this solution at 0°C were added (2*S*)-pyrrolidine-2-carbonitrile hydrochloride (270mg, 2.04mmol) and PyBOP® (1.3g, 2.49mmol), and the pH adjusted to pH9 with triethylamine. After 18h at 0°C to room temperature the solvent was removed *in vacuo* and the residue was taken up in ethyl acetate (150mL). The solution was washed with 0.3M KHSO₄ (2 x 30mL), sat. NaHCO₃ (2 x 30mL), water (2 x 30mL) and brine (1 x 30mL), dried (Na₂SO₄) and evaporated *in vacuo*. The residue was purified by flash chromatography (eluant: 75% ethyl acetate, 25% pet. ether) to give a colourless oil identified as (2*S*)-1-[(2'*R*)-3'-(acetylaminomethylthio)-2'-(*tert*-butyloxycarbonylamino)-3'-methylbutanoyl]pyrrolidine-2-carbonitrile (742mg, 1.86mmol, 83%).

### B. (2S)-1-[(2'R)-3'-(Acetylaminomethylthio)-2'-amino-3'-methylbutanoyl]pyrrolidine-2-carbonitrile trifluoroacetate

(2*S*)-1-[(2'*R*)-3'-(Acetylaminomethylthio)-2'-(*tert*-butyloxycarbonylamino)-3'-methyl-butanoyl]pyrrolidine-2-carbonitrile (710mg, 1.78mmol) was dissolved in trifluoroacetic acid (30mL). After 1 h at room temperature the solvent was removed *in vacuo* to give a colourless oil identified as (2*S*)-1-[(2'*R*)-3'-(acetylaminomethylthio)-2'-amino-3'-methyl-butanoyl]pyrrolidine-2-carbonitrile trifluoroacetate (560mg, 1.36mmol, 76%).
[M+H]⁺ = 299.1

### EXAMPLE 9

### (2S)-1-[N^{ω}-(2-Chloropyridyl-3-carbonyl)-L-ornithinyl]pyrrolidine-2-carbonitrile trifluoroacetate

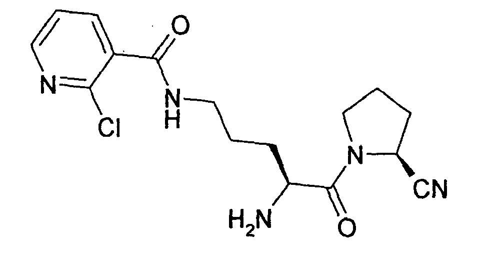

### A. (2S)-1-(N^{α}-(tert-Butyloxycarbonyl)-L-ornithyl)pyrrolidine-2-carbonitrile

(2*S*)-1-(*N*^{α}-(*tert*-Butyloxycarbonyl)-L-ornithyl)pyrrolidine-2-carbonitrile was prepared by the method described for the lysine derivative in Example 2.

### B. (2S)-1-(N^{α}-(tert-Butyloxycarbonyl)-N^{ω}-(2-chloropyridyl-3-carbonyl)-L-ornithyl)-pyrrolidine-2-carbonitrile

(2*S*)-1-(*N*^{α}-(*tert*-Butyloxycarbonyl)-L-omithyl)pyrrolidine-2-carbonitrile (80mg, 0.26mmol) was dissolved in CH₂Cl₂ (20mL). To this solution was added 2-chloropyridine-3-carbonyl chloride (55mg, 0.32mmol) and the pH adjusted to pH9 with triethylamine. After 18h at room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo*. The residue was purified by flash chromatography (eluant: 95% ethyl acetate, 5% pet. ether) to give a colourless oil identified as (2*S*)-1-(*N*^{α}-(*tert*-butyloxycarbonyl)-*N*^{ω}-(2-chloropyridyl-3-carbonyl)-L-ornithyl)pyrrolidine-2-carbonitrile (60mg, 0.14mmol, 53%).

### C. (2S)-1-[N^{ω}-(2-Chloropyridyl-3-carbonyl)-L-ornithinyl]pyrrolidine-2-carbonitrile trifluoroacetate

(2*S*)-1-[*N*^{α}-(*tert*-Butyloxycarbonyl)-*N*^{ω}-(2-chloropyridyl-3-carbonyl)-L-ornithinyl]pyrrolidine-2-carbonitrile (60mg, 0.14mmol) was dissolved in trifluoroacetic acid (5mL). After 1h at room temperature the solvent was removed *in vacuo.* The residue was purified by preparative hplc (Vydac C18, 5 to 50% 0.1% TFA/acetonitrile into 0.1% TFA/water over 40min at 3mUmin). Fractions containing the product were lyophilised to give a white solid identified as (2*S*)-1-[*N*^{ω}-(2-chloropyridyl-3-carbonyl)-L-ornithinyl]pyrrolidine-2-carbonitrile trifluoroacetate (52mg).
[M+H]⁺ = 350.1

### EXAMPLE 10

### 1-[N^{ω}-(2-Chloropyridyl-3-carbonyl)-L-ornithinyl]pyrrolidine hydrochloride

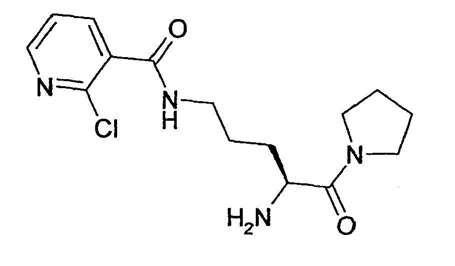

### A. 1-(N^{α}-(tert-Butyloxycarbonyl)-N^{ω}-(2-chloropyridyl-3-carbonyl)-L-ornithyl)-pyrrolidine

1-(*N*^{α}-(*tert*-Butyloxycarbonyl)-L-ornithyl)pyrrolidine (20mg, 0.069mmol) was dissolved in CH₂Cl₂ (5mL). To this solution was added 2-chloropyridine-3-carbonyl chloride (14mg, 0.076mmol) and the pH adjusted to pH9 with triethylamine. After 1h at room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo*. The residue was purified by flash chromatography (eluant: 10% methanol, 90% dichloromethane) to give a colourless oil identified as 1-(*N*^{α}-(*tert*-butyloxycarbonyl)-*N*^{ω}-(2-chloropyridyl-3-carbonyl)-L-omithyl)pyrrolidine (19mg, 0.045mmol, 63%).

### B. 1-[N^{ω}-(2-Chloropyridyl-3-carbonyl)-L-omithinyl]pyrrolidine hydrochloride

1-[*N*^{α}-(*tert*-Butyloxycarbonyl)-*N*^{ω}-(2-chloropyridyl-3-carbonyl)-L-omithinyl]pyrrolidine (19mg, 0.045mmol) was dissolved in 4M HCl/dioxan (10mL). After 45min at room temperature the solvent was removed *in vacuo* to give a white solid identified as 1-[*N*^{ω}-(2-chloropyridyl-3-carbonyl)-L-omithinyl]pyrrolidine hydrochloride (15mg).
[M+H]⁺ = 325.1

### EXAMPLE 11

### 3-[N^{ω}-(2-Chloropyridyl-3-carbonyl)-L-ornithinyl]thiazolidine hydrochloride

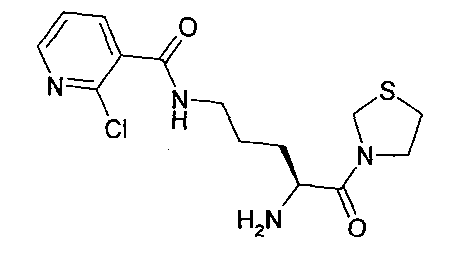

### A. 3-(N^{α}-(tert-Butyloxycarbonyl)-N^{ω}-(2-chloropyridyl-3-carbonyl)-L-ornithyl)-thiazolidine

3-(*N*^{α}-(*tert*-Butyloxycarbonyl)-L-ornithyl)thiazolidine (136mg, 0.45mmol) was dissolved in CH₂Cl₂ (10mL). To this solution was added 2-chloropyridine-3-carbonyl chloride (88mg, 0.5mmol) and the pH adjusted to pH9 with triethylamine. After 1 h at room temperature the solvent was removed *in vacuo* and the residue was taken up in ethyl acetate (70mL). The solution was washed with 0.3M KHSO₄ (2 x 20mL), sat. NaHCO₃ (2 x 20mL), water (2 x 20mL) and brine (1 x 20mL), dried (Na₂SO₄) and evaporated *in vacuo*. The residue was purified by flash chromatography (eluant: 1.5% methanol, 98.5% dichloromethane) to give a colourless oil identified as 3-(*N*^{α}-(*tert*-butyloxycarbonyl)-*N*^{ω}-(2-chloropyridyl-3-carbonyl)-L-ornithyl)thiazolidine (30mg, 0.068mmol, 15%).

### B. 3-[N^{ω}-(2-Chloropyridyl-3-carbonyl)-L-ornithinyl]thiazolidine hydrochloride

3-[*N*^{α}-(*tert*-Butyloxycarbonyl)-*N*^{ω}-(2-chloropyridyl-3-carbonyl)-L-ornithinyl]thiazolidine (30mg, 0.068mmol) was dissolved in 4M HCl/dioxan (10mL). After 45min at room temperature the solvent was removed *in vacuo* to give a white solid identified as 1-[*N*^{ω}-(2-chloropyridyl-3-carbonyl)-L-ornithinyl]thiazolidine hydrochloride (25mg).
[M+H]⁺ = 342.1

### EXAMPLE 12

### (2S)-1-[S-(3-Picolylcarbamoylmethyl)-L-cysteinyl]pyrrolidine-2-carbonitrile trifluoroacetate

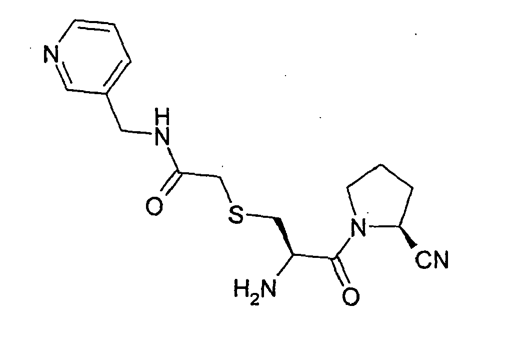

### A. S-(Benzyloxycarbonylmethyl)-N-(tert-butyloxycarbonyl)-L-cysteine

*N*-(*tert*-Butyloxycarbonyl)-L-cysteine (3.5g, 15.8mmol), benzyl 2-bromoacetate (3.7g, 16.1mmol) and triethylamine (1.8g, 18.0mmol) were dissolved in THF (100mL). After 18 hours at room temperature the reaction mixture was diluted with ethyl acetate (100mL), washed with 0.3M KHSO₄, sat NaHCO₃, water and brine, dried (Na₂SO₄) and evaporated. The residue was purified by flash chromatography (eluant 95% chloroform, 4% methanol, 1% acetic acid) yielding a colourless oil identified as *S*-(benzyloxycarbonylmethyl)-*N*-(*tert-*butyloxycarbonyl)-L-cysteine (5.2g, 14.1mmol, 89%).

### B. (2S)-1-[S-(Benzyloxycarbonylmethyl)-N-(tert-butyloxycarbonyl)-L-cysteinyl]-pyrrolidine-2-carbonitrile

*S*-(Benzyloxycarbonylmethyl)-*N*-(*tert*-butyloxycarbonyl)-L-cysteine (5.10g, 13.8mmol) was dissolved in CH₂Cl₂ (200mL). This solution was cooled to 0°C, (2*S*)-pyrrolidine-2-carbonitrile hydrochloride (2.1g, 15.8mmol) and PyBOP (8.0g, 15.3mmol) were added, and the pH was adjusted to pH9 with triethylamine. After 18h at 0°C to room temperature the solvent was removed *in vacuo* and the residue was taken up in ethyl acetate (150mL). This solution was washed with 0.3M KHSO₄ (1 x 50mL), sat. NaHCO₃ (1 x 50mL), water (1 x 50mL) and brine (1 x 50mL), dried (Na₂SO₄) and evaporated *in vacuo* to give a yellow oil. This was purified by flash chromatography (eluant: 40% ethyl acetate, 60% pet. ether) to give a colourless oil identified as (2*S*)-1-[*S*-(benzyloxycarbonylmethyl)-*N*-(*tert-*butyloxycarbonyl)-L-cysteinyl]pyrrolidine-2-carbonitrile (5.82g, 13.0mmol, 94%).

### C. (2S)-1-[N-(tert-Butyloxycarbonyl)-S-(carboxymethyl)-L-cysteinyl]pyrrolidine-2-carbonitrile

(2*S*)-1-[*S*-(Benzyloxycarbonylmethyl)-*N*-(*tert*-butyloxycarbonyl)-L-cysteinyl]pyrrolidine-2-carbonitrile (1.31g, 2.9mmol) was dissolved in THF(100mL). Aqueous lithium hydroxide (1 M, 3.5mL, 3.5mmol) was added. After 3 hours at room temperature the reaction mixture was diluted with ethyl acetate (100mL), washed with 1 M citric acid, water and brine, dried (Na₂SO₄) and evaporated *in vacuo* to give a colourless oil. This was purified by flash chromatography (eluant: 97% chloroform, 2% methanol, 1% acetic acid) to give a colourless oil identified as (2*S*)-1-[*N*-(*tert*-butyloxycarbonyl)-*S*-(carboxymethyl)-L-cysteinyl]pyrrolidine-2-carbonitrile (860mg, 2.4mmol, 82%).

### D. (2S)-1- [N-(tert-Butyloxycarbonyl)-S-(3-picolylcarbamoylmethyl)-L-cysteinyl]-pyrrolidine-2-carbonitrile

(2*S*)-1-[*N*-(*tert*-Butyloxycarbonyl)-*S*-(carboxymethyl)-L-cysteinyl]pyrrolidine-2-carbonitrile (150mg, 0.42mmol) was dissolved in CH₂Cl₂ (20mL). This solution was cooled to 0°C, 3-(aminomethyl)pyridine (53mg, 0.5mmol) and PyBOP (270mg, 0.52mmol) were added, and the pH adjusted to pH9 with triethylamine. After 18h at 0°C to room temperature the solvent was removed *in vacuo* and the residue was taken up in ethyl acetate (70mL). This solution was washed with 0.3M KHSO₄ (1 x 20mL), sat. NaHCO₃ (1 x 20mL), water (1 x 20mL) and brine (1 x 20mL), dried (Na₂SO₄) and evaporated *in vacuo* to give a yellow oil. This was purified by flash chromatography (eluant: 96% chloroform, 4% methanol) to give a colourless oil identified as (2*S*)-1-[*N*-(*tert*-butyloxycarbonyl)-*S*-(3-picolylcarbamoylmethyl)-L-cysteinyl]pyrrolidine-2-carbonitrile (170mg, 0.38mmol, 91%).

### E. (2S)-1-[S-(3-Picolylcarbamoylmethyl)-L-cysteinyl]pyrrolidine-2-carbonitrile trifluoroacetate

(2*S*)-1-[*N*-(*tert*-Butyloxycarbonyl)-*S*-(3-picolylcarbamoylmethyl)-L-cysteinyl]pyrrolidine-2-carbonitrile (130mg, 0.29mmol) was dissolved in trifluoroacetic acid (10mL). After 1 hour at room temperature the solvent was removed *in vacuo* to give a white solid identified as (2*S*)-1-[*S*-(3-picolylcarbamoylmethyl)-L-cysteinyl]pyrrolidine-2-carbonitrile trifluoroacetate (116mg,0.25mmol, 86%).
[M+H]⁺ = 348.2

### EXAMPLE 13

### 3-[N^{ω}-(2-Quinoxaloyl)-L-lysinyl]thiazolidine hydrochloride

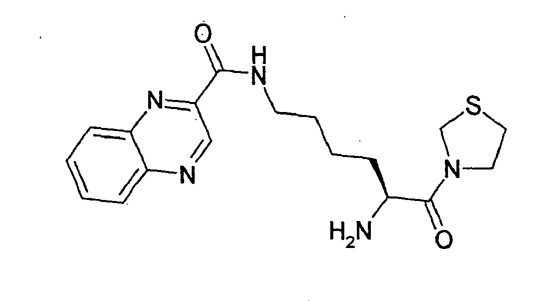

### A. 3-[N^{α}-(tert-Butyloxycarbonyl)-N^{ω}-(2-quinoxaloyl)-L-lysinyl]thiazolidine

3-[*N*^{α}-(*tert*-Butyloxycarbonyl)lysinyl]thiazolidine (128mg, 0.4mmol) was dissolved in CH₂Cl₂ (10mL). 2-Quinoxaloyl chloride (85mg, 0.44mmol) and potassium carbonate (45.8mg, 0.3mmol) were added. The reaction mixture was stirred at room temperature for 18 hours and the solvent removed *in vacuo*. The residue was purified by flash chromatography (eluant: 99.5% chloroform, 0.5% methanol) to give a colourless oil identified as 3-[*N*^{α}-(*tert-*butyloxycarbonyl)-*N*^{ω}-(2-quinoxaloyl)-L-lysinyl]thiazolidine (140mg, 0.296mmol, 74%).

### B. 3-[N^{ω}-(2-Quinoxaloyl)-L-lysinyl]thiazolidine hydrochloride

3-[*N*^{α}-(*tert*-Butyloxycarbonyl)-*N*^{ω}-(2-quinoxaloyl)-L-lysinyl]thiazolidine (140mg, 0.296mmol) was dissolved in 4M HCl/dioxan (20mL). After 1 hour at room temperature the solvent was removed *in vacuo* to give a white solid identified as 3-[*N*^{ω}-(2-quinoxaloyl)-L-lysinyl]thiazolidine hydrochloride (128mg, 0.296mmol, 100%).
[M+H]⁺ = 374.2

### EXAMPLE 14

### 3-[N^{ω}-(3-Pyridyloxycarbonyl)-L-ornithinyl]thiazolidine hydrochloride

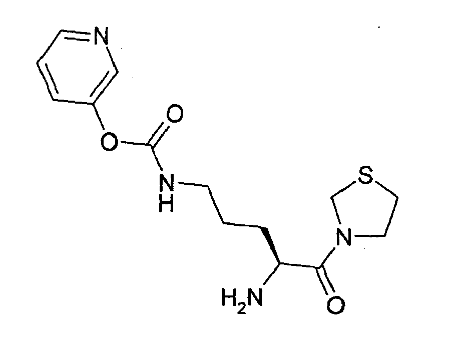

### A. 3-[N^{α}-(tert-Butyloxycarbonyl)-N^{ω}-(3-pyridyloxycarbonyl)-L-ornithinyl]thiazolidine

3-Hydroxypyridine (14.9mg, 0.138mmol) was dissolved in CH₂Cl₂ (20mL). Phosgene (20% solution in toluene, 0.335mL, 0.685mmol) and pyridine (14mg, 0.182mmol) were added at 0°C. After 90 mins the solvent was removed *in vacuo* and the residue dissolved in CH₂Cl₂ (20mL). 3-[*N*^{α}-(*tert*-Butyloxycarbonyl)ornithinyl]thiazolidine (42mg, 0.138mmol) and triethylamine (28mg, 0.28mmol) were added. The reaction mixture was stirred at room temperature for 18 hours and the solvent removed *in vacuo*. The residue was purified by flash chromatography (eluant: 97% chloroform, 3% methanol) to give a colourless oil identified as 3-[*N*^{α}-(*tert*-butyloxycarbonyl)-*N*^{ω}-(3-pyridyloxycarbonyl)-L-ornithinyl]thiazolidine (16mg, 0.038mmol, 27%).

### B. 3-[N^{ω}-(3-Pyridyloxycarbonyl)-L-ornithinyl]thiazolidine hydrochloride

3-[*N*^{α}-(*tert*-Butyloxycarbonyl)-*N*^{ω}-(3-pyridyloxycarbonyl)-L-omithinyl]thiazolidine (16mg, 0.038mmol) was dissolved in 4M HCl/dioxan (20mL). After 1 hour at room temperature the solvent was removed *in vacuo* to give a white solid identified as 3-[*N*^{ω}-(3-pyridyloxycarbonyl)-L-omithinyl]thiazolidine hydrochloride (14mg, 0.038mmol, 100%).
[M+H]⁺ = 325.1

### EXAMPLE 15

### 3-[O-(3-Chlorobenzylcarbamoyl)serinyl]thiazolidine hydrochloride

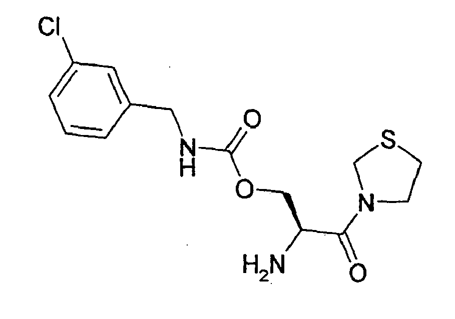

### A. 3-[N-(tert-Butyloxycarbonyl)-L-serinyl]thiazolidine

*N*-(*tert*-Butyloxycarbonyl)-L-serine (2.1g, 10.2mmol) was dissolved in CH₂Cl₂/DMF (9:1,50mL). Thiazolidine (650mg, 11.2mmol), hydroxybenzotriazole(2.8g, 20.7mmol) and water soluble carbodiimide (3.9g, 19.5mmol) were added at 0°C. The pH was adjusted to pH8 with *N*-methylmorpholine. The reaction mixture was stirred at room temperature for 18 hours, the solvent removed *in vacuo* and the residue was taken up in ethyl acetate (150mL). This solution was washed with 0.3M KHSO₄ (1 x 50mL), sat. NaHCO₃ (1 x 50mL), water (1 x 50mL) and brine (1 x 50mL), dried (Na₂SO₄) and evaporated *in vacuo* to give a white solid identified as 3-[*N*-(*tert*-butyloxycarbonyl)-L-serinyl]thiazolidine (2.15g, 7.78mmol, 76%).

### B. 3-[N-(tert-Butyloxycarbonyl)-O-(3-chlorobenzylcarbamoyl)-L-serinyl]thiazolidine

3-[*N*-(*tert*-Butyloxycarbonyl)-L-serinyl]thiazolidine (110mg, 0.48mmol) was dissolved in DMF (10mL) and 1,1'-carbonyl-diimidazole (71mg, 0.43mmol) was added. After 2 hours at room temperature 3-chlorobenzylamine (62mg, 0.4mmol) was added. After a further 18 hours EtOAc (200mL) was added. This solution was washed with 0.3M KHSO₄ (1 x 50mL), sat. NaHCO₃ (1 x 50mL), water (4 x 50mL) and brine (1 x 50mL), dried (Na₂SO₄) and evaporated *in vacuo* to give a yellow oil. This was purified by flash chromatography (eluant: 40% ethyl acetate, 60% pet. ether) to give a colourless oil identified as 3-[*N*-(*tert-*butyloxycarbonyl)-*O*-(3-chlorobenzylcarbamoyl)-L-serinyl]thiazolidine (158mg, 0.36mmol, 90%).

### C. 3-[O-(3-Chlorobenzylcarbamoyl)-L-serinyl]thiazolidine hydrochloride

3-[*N*-(*tert*-Butyloxycarbonyl)-O-(3-chlorobenzylcarbamoyl)-L-serinyl]thiazolidine (140mg, 0.32mmol) was dissolved in 4M HCl/dioxan (20mL). After 1 hour at room temperature the solvent was removed *in vacuo* to give a white solid identified as 3-[*O*-(3-chlorobenzylcarbamoyl)-L-serinyl]thiazolidine hydrochloride (115mg, 0.3mmol, 94%).
[M+H]⁺ = 344.1

The Examples set out in the following Tables were prepared by analogous methods to the above.

**TABLE 1 - Examples 16 - 162**

| | | | | |
|---|---|---|---|---|
| 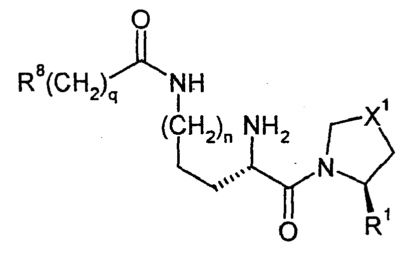 | | | | |

| **Example** | **n** | **R**^{**8**}**(CH**_{**2**}**)**_{**q**} | **R**^{**1**} | **X**^{**1**} |
|---|---|---|---|---|
| **16** | 1 | 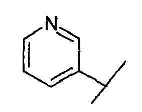 | CN | CH₂ |
| **17** | 2 | | CN | CH₂ |
| **18** | 1 | | H | S |
| **19** | 2 | | H | S |
| **20** | 1 | 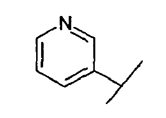 | CN | S |
| **21** | 2 | | CN | S |
| **22** | 1 | | H | CH₂ |
| **23** | 2 | | H | CH₂ |
| **24** | 1 | 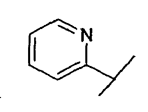 | CN | CH₂ |
| **25** | 2 | | CN | CH₂ |
| **26** | 1 | | H | S |
| **27** | 2 | | H | S |
| **28** | 1 | 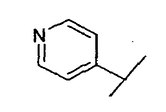 | CN | CH₂ |
| **29** | 2 | | CN | CH₂ |
| **30** | 1 | | H | S |
| **31** | 2 | | H | S |
| **32** | 1 | 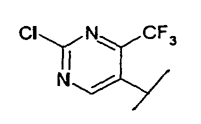 | CN | CH₂ |
| **33** | 2 | | CN | CH₂ |
| **34** | 1 | | H | S |
| **35** | 2 | | H | S |
| **36** | 1 | 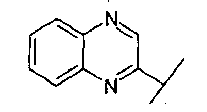 | CN | CH₂ |
| **37** | 2 | | CN | CH₂ |
| **38** | 1 | | H | S |
| **39** | 1 | 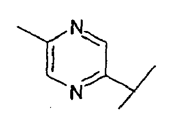 | CN | CH₂ |
| **40** | 2 | | CN | CH₂ |
| **41** | 1 | | H | S |
| **42** | 2 | | H | S |
| **43** | 1 | 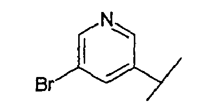 | CN | CH₂ |
| **44** | 2 | | CN | CH₂ |
| **45** | 1 | | H | S |
| **46** | 2 | | H | S |
| **47** | 1 | 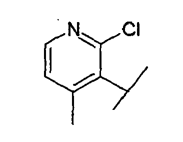 | CN | CH₂ |
| **48** | 2 | | CN | CH₂ |
| **49** | 1 | | H | S |
| **50** | 2 | | H | S |
| **51** | 1 | 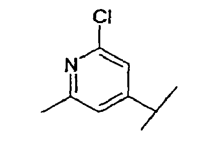 | CN | CH₂ |
| **52** | 2 | | CN | CH₂ |
| **53** | 1 | | H | S |
| **54** | 2 | | H | S |
| **55** | 1 | 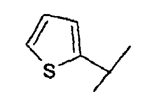 | CN | CH₂ |
| **56** | 2 | | CN | CH₂ |
| **57** | 1 | | H | S |
| **58** | 2 | | H | S |
| **59** | 1 | 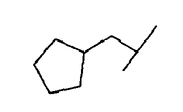 | CN | CH₂ |
| **60** | 2 | | CN | CH₂ |
| **61** | 1 | | H | S |
| **62** | 2 | | H | S |
| **63** | 1 | 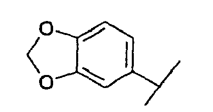 | CN | CH₂ |
| **64** | 2 | | CN | CH₂ |
| **65** | 1 | | H | S |
| **66** | 2 | | H | S |
| **67** | 1 | 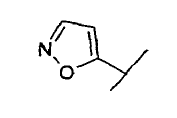 | CN | CH₂ |
| **68** | 2 | | CN | CH₂ |
| **69** | 1 | | H | S |
| **70** | 2 | | H | S |
| **71** | 1 | 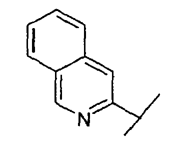 | CN | CH₂ |
| **72** | 2 | | CN | CH₂ |
| **73** | 1 | | H | S |
| **74** | 2 | | H | S |
| **75** | 1 | 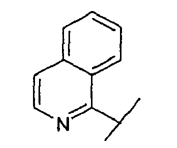 | CN | CH₂ |
| **76** | 2 | | CN | CH₂ |
| **77** | 1 | | H | S |
| **78** | 2 | | H | S |
| **79** | 1 | 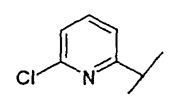 | CN | CH₂ |
| **80** | 2 | | CN | CH₂ |
| **81** | 1 | | H | S |
| **82** | 2 | | H | S |
| **83** | 1 | 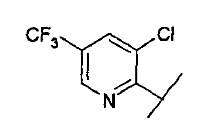 | CN | CH₂ |
| **84** | 2 | | CN | CH₂ |
| **85** | 1 | | H | S |
| **86** | 2 | | H | S |
| **87** | 1 | 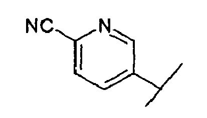 | CN | CH₂ |
| **88** | 2 | | CN | CH₂ |
| **89** | 1 | | H | S |
| **90** | 2 | | H | S |
| **91** | 1 | 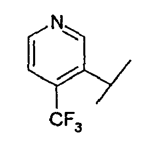 | CN | CH₂ |
| **92** | 2 | | CN | CH₂ |
| **93** | 1 | | H | S |
| **94** | 2 | | H | S |
| **95** | 1 | 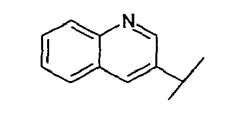 | CN | CH₂ |
| **96** | 2 | | CN | CH₂ |
| **97** | 1 | | H | S |
| **98** | 2 | | H | S |
| **99** | 1 | 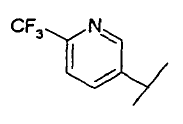 | CN | CH₂ |
| **100** | 2 | | CN | CH₂ |
| **101** | 1 | | H | S |
| **102** | 2 | | H | S |
| **103** | 1 | 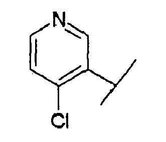 | CN | CH₂ |
| **104** | 2 | | CN | CH₂ |
| **105** | 1 | | H | S |
| **106** | 2 | | H | S |
| **107** | 1 | 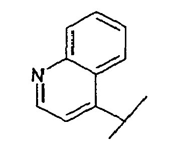 | CN | CH₂ |
| **108** | 2 | | CN | CH₂ |
| **109** | 1 | | H | S |
| **110** | 2 | | H | S |
| **111** | 1 | 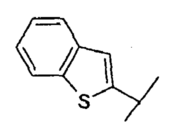 | CN | CH₂ |
| **112** | 2 | | CN | CH₂ |
| **113** | 1 | | H | S |
| **114** | 2 | | H | S |
| **115** | 1 | 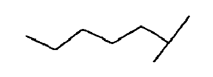 | CN | CH₂ |
| **116** | 2 | | CN | CH₂ |
| **117** | 1 | | H | S |
| **118** | 2 | | H | S |
| **119** | 1 | 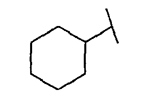 | CN | CH₂ |
| **120** | 2 | | CN | CH₂ |
| **121** | 1 | | H | S |
| **122** | 2 | | H | S |
| **123** | 1 | 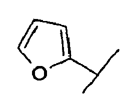 | CN | CH₂ |
| **124** | 2 | | CN | CH₂ |
| **125** | 1 | | H | S |
| **126** | 2 | | H | S |
| **127** | 1 | 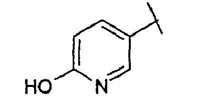 | H | S |
| **128** | 1 | 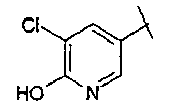 | H | S |
| **129** | 2 | | H | S |
| **130** | 1 | 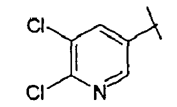 | H | S |
| **131** | 2 | | H | S |
| **132** | 1 | 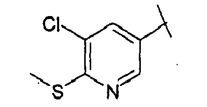 | H | S |
| **133** | 1 | 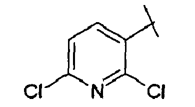 | H | S |
| **134** | 1 | 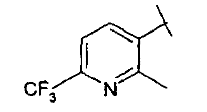 | H | S |
| **135** | 1 | 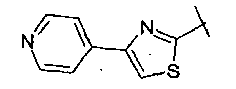 | H | S |
| **136** | 1 | 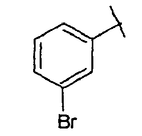 | H | S |
| **137** | 1 | 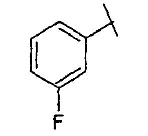 | H | S |
| **138** | 1 | 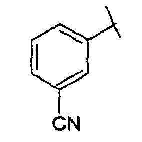 | H | S |
| **139** | 1 | 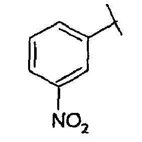 | H | S |
| **140** | 1 | 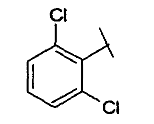 | H | S |
| **141** | 1 | 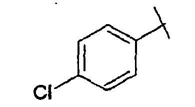 | H | S |
| **142** | 1 | 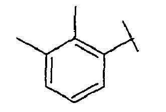 | H | S |
| **143** | 1 | 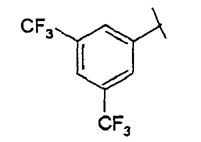 | H | S |
| **144** | 1 | 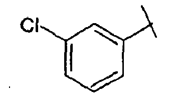 | H | CH₂ |
| **145** | 1 | 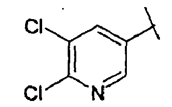 | H | CH₂ |
| **146** | 1 | 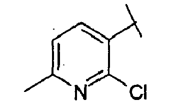 | H | CH₂ |
| **147** | 1 | 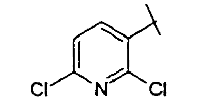 | H | CH₂ |
| **148** | 1 | 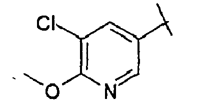 | H | S |
| **149** | 1 | 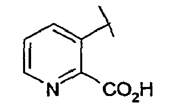 | H | S |
| **150** | 2 | | H | S |
| **151** | 1 | 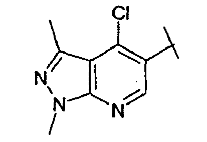 | H | S |
| **152** | 2 | | H | S |
| **153** | 1 | 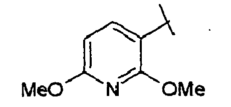 | H | S |
| **154** | 1 | 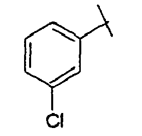 | H | S |
| **155** | 1 | 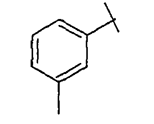 | H | S |
| **156** | 1 | 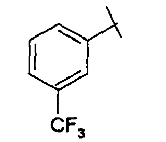 | H | S |
| **157** | 1 | 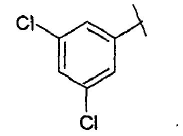 | H | S |
| **158** | 1 | 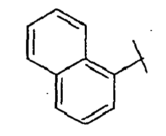 | H | S |
| **159** | 1 | 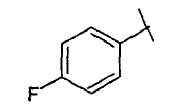 | H | S |
| **160** | 1 | 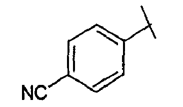 | H | S |
| **161** | 1 | 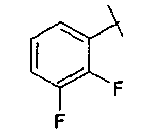 | H | S |
| **162** | 1 | 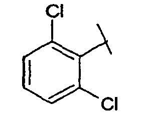 | H | CH₂ |

**TABLE 2 - Examples 163 - 250**

| | | | | |
|---|---|---|---|---|
| 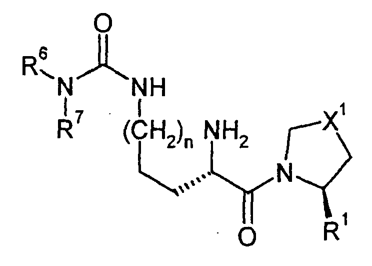 | | | | |

| **Example** | **n** | **R**^{**6**}**R**^{**7**}**N** | **R**^{**1**} | **X**^{**1**} |
|---|---|---|---|---|
| **163** | 1 | 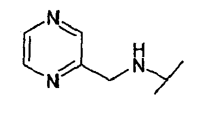 | CN | CH₂ |
| **164** | 2 | | CN | CH₂ |
| **165** | 1 | | H | S |
| **166** | 2 | | H | S |
| **167** | 1 | 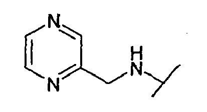 | CN | S |
| **168** | 2 | | CN | S |
| **169** | 1 | | H | CH₂ |
| **170** | 2 | | H | CH₂ |
| **171** | 1 | 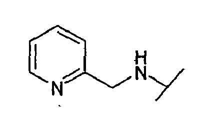 | CN | CH₂ |
| **172** | 2 | | CN | CH₂ |
| **173** | 1 | | H | S |
| **174** | 2 | | H | S |
| **175** | 1 | 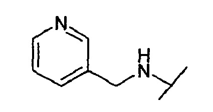 | CN | CH₂ |
| **176** | 2 | | CN | CH₂ |
| **177** | 1 | | H | S |
| **178** | 2 | | H | S |
| **179** | 1 | 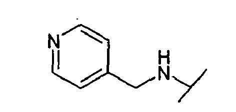 | CN | CH₂ |
| **180** | 2 | | CN | CH₂ |
| **181** | 1 | | H | S |
| **182** | 2 | | H | S |
| **183** | 1 | 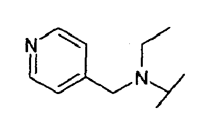 | CN | CH₂ |
| **184** | 2 | | CN | CH₂ |
| **185** | 1 | | H | S |
| **186** | 2 | | H | S |
| **187** | 1 | 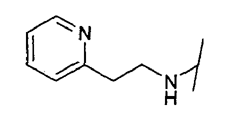 | CN | CH₂ |
| **188** | 2 | | CN | CH₂ |
| **189** | 1 | | H | S |
| **190** | 2 | | H | S |
| **191** | 1 | NH₂ | CN | CH₂ |
| **192** | 2 | | CN | CH₂ |
| **193** | 1 | | H | S |
| **194** | 2 | | H | S |
| **195** | 1 | 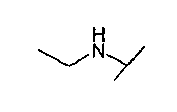 | CN | CH₂ |
| **196** | 2 | | CN | CH₂ |
| **197** | 1 | | H | S |
| **198** | 2 | | H | S |
| **199** | 1 | 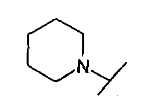 | CN | CH₂ |
| **200** | 2 | | CN | CH₂ |
| **201** | 1 | | H | S |
| **202** | 2 | | H | S |
| **203** | 1 | 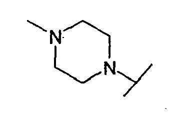 | CN | CH₂ |
| **204** | 2 | | CN | CH₂ |
| **205** | 1 | | H | S |
| **206** | 2 | | H | S |
| **207** | 1 | 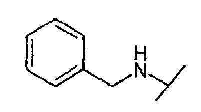 | CN | CH₂ |
| **208** | 2 | | CN | CH₂ |
| **209** | 1 | | H | S |
| **210** | 2 | | H | S |
| **211** | 1 | 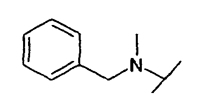 | CN | CH₂ |
| **212** | 2 | | CN | CH₂ |
| **213** | 1 | | H | S |
| **214** | 2 | | H | S |
| **215** | 1 | 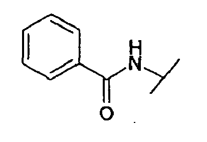 | CN | CH₂ |
| **216** | 2 | | CN | CH₂ |
| **217** | 1 | | H | S |
| **218** | 2 | | H | S |
| **219** | 1 | 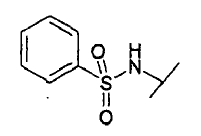 | CN | CH₂ |
| **220** | 2 | | CN | CH₂ |
| **221** | 1 | | H | S |
| **222** | 2 | | H | S |
| **223** | 1 | 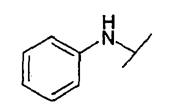 | CN | CH₂ |
| **224** | 2 | | CN | CH₂ |
| **225** | 1 | | H | S |
| **226** | 2 | | H | S |
| **227** | 1 | 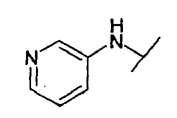 | CN | CH₂ |
| **228** | 2 | | CN | CH₂ |
| **229** | 1 | | H | S |
| **230** | 2 | | H | S |
| **231** | 1 | 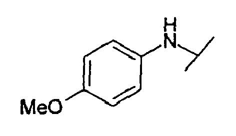 | CN | CH₂ |
| **232** | 2 | | CN | CH₂ |
| **233** | 1 | | H | S |
| **234** | 2 | | H | S |
| **235** | 1 | 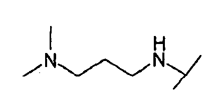 | CN | CH₂ |
| **236** | 2 | | CN | CH₂ |
| **237** | 1 | | H | S |
| **238** | 2 | | H | S |
| **239** | 1 | 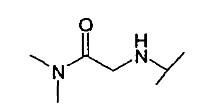 | CN | CH₂ |
| **240** | 2 | | CN | CH₂ |
| **241** | 1 | | H | S |
| **242** | 2 | | H | S |
| **243** | 1 | 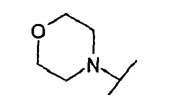 | CN | CH₂ |
| **244** | 2 | | CN | CH₂ |
| **245** | 1 | | H | S |
| **246** | 2 | | H | S |
| **247** | 1 | 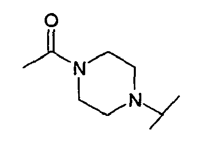 | CN | CH₂ |
| **248** | 2 | | CN | CH₂ |
| **249** | 1 | | H | S |
| **250** | 2 | | H | S |

**TABLE 3 - Examples 251 - 266**

| | | | | |
|---|---|---|---|---|
| 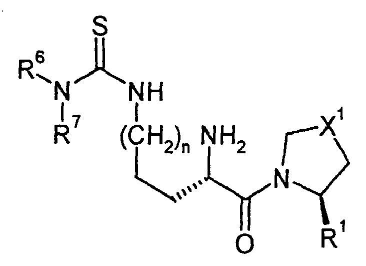 | | | | |

| **Example** | **n** | **R**^{**6**}**R**^{**7**}**N** | **R**^{**1**} | **X**^{**1**} |
|---|---|---|---|---|
| **251** | 1 | 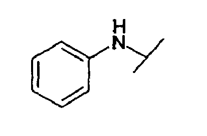 | CN | CH2 |
| **252** | 2 | | CN | CH₂ |
| **253** | 1 | | H | S |
| **254** | 2 | | H | S |
| **255** | 1 | 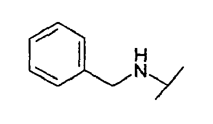 | CN | CH₂ |
| **256** | 2 | | CN | CH₂ |
| **257** | 1 | | H | S |
| **258** | 2 | | H | S |
| **259** | 1 | 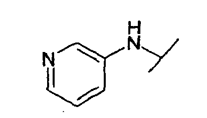 | CN | CH₂ |
| **260** | 2 | | CN | CH₂ |
| **261** | 1 | | H | S |
| **262** | 2 | | H | S |
| **263** | 1 | 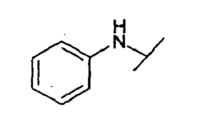 | CN | S |
| **264** | 2 | | CN | S |
| **265** | 1 | | H | CH₂ |
| **266** | 2 | | H | CH₂ |

**TABLE 4 - Examples 267 - 318**

| | | | | |
|---|---|---|---|---|
| 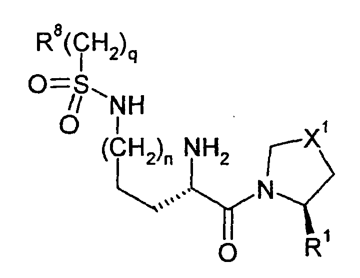 | | | | |

| **Example** | **n** | **R**^{**8**}**(CH**_{**2**}**)**_{**q**} | **R**^{**1**} | **X**^{**1**} |
|---|---|---|---|---|
| **267** | 1 | 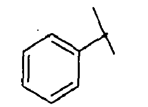 | CN | CH₂ |
| **268** | 2 | | CN | CH₂ |
| **269** | 1 | 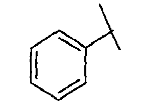 | CN | S |
| **270** | 2 | | CN | S |
| **271** | 1 | 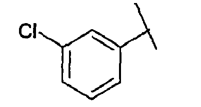 | CN | CH₂ |
| **272** | 2 | | CN | CH₂ |
| **273** | 1 | 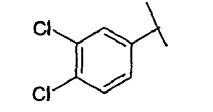 | CN | CH₂ |
| **274** | 2 | | CN | CH₂ |
| **275** | 1 | 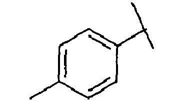 | CN | CH₂ |
| **276** | 2 | | CN | CH₂ |
| **277** | 1 | 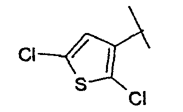 | CN | CH₂ |
| **278** | 2 | | CN | CH₂ |
| **279** | 1 | 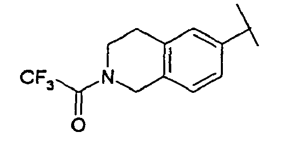 | CN | CH₂ |
| **280** | 2 | | CN | CH₂ |
| **281** | 1 | 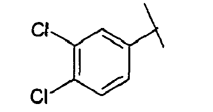 | H | CH₂ |
| **282** | 2 | | H | CH₂ |
| **283** | 1 | 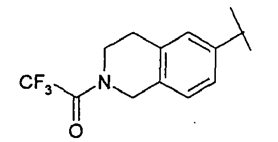 | H | CH₂ |
| **284** | 2 | | H | CH₂ |
| **285** | 1 | 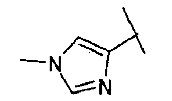 | H | CH₂ |
| **286** | 2 | | H | CH₂ |
| **287** | 1 | 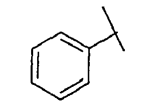 | H | S |
| **288** | 2 | | H | S |
| **289** | 1 | 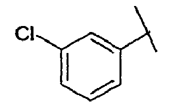 | H | S |
| **290** | 2 | | H | S |
| **291** | 1 | 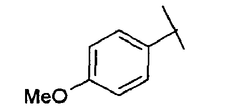 | H | S |
| **292** | 2 | | H | S |
| **293** | 1 | 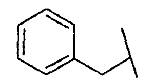 | CN | CH₂ |
| **294** | 2 | | CN | CH₂ |
| **295** | 1 | 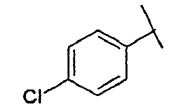 | CN | CH₂ |
| **296** | 2 | | CN | CH₂ |
| **297** | 1 | 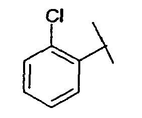 | CN | CH₂ |
| **298** | 2 | | CN | CH₂ |
| **299** | 1 | 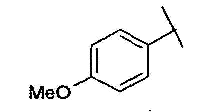 | CN | CH₂ |
| **300** | 2 | | CN | CH₂ |
| **301** | 1 | 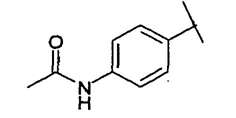 | CN | CH₂ |
| **302** | 2 | | CN | CH₂ |
| **303** | 1 | 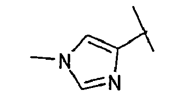 | CN | CH₂ |
| **304** | 2 | | CN | CH₂ |
| **305** | 1 | 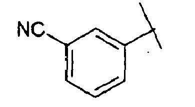 | CN | CH₂ |
| **306** | 2 | | CN | CH₂ |
| **307** | 1 | 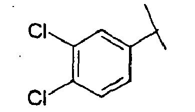 | H | S |
| **308** | 2 | | H | S |
| **309** | 1 | 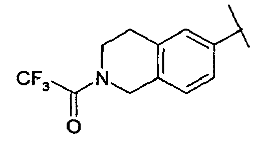 | H | S |
| **310** | 2 | | H | S |
| **311** | 1 | 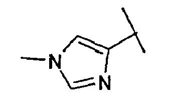 | H | S |
| **312** | 2 | | H | S |
| **313** | 1 | 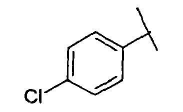 | H | S |
| **314** | 2 | | H | S |
| **315** | 1 | 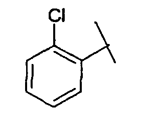 | H | S |
| **316** | 2 | | H | S |
| **317** | 1 | 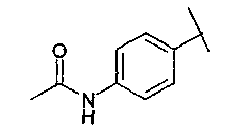 | H | S |
| **318** | 2 | | H | S |

**TABLE 5 - Examples 319 - 378**

| | | | |
|---|---|---|---|
| 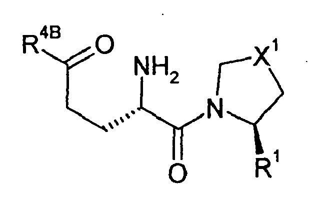 | | | |

| **Example** | **R**^{**4B**} | **R**^{**1**} | **X**^{**1**} |
|---|---|---|---|
| **319** | NH₂ | CN | CH₂ |
| **320** | 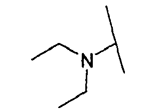 | CN | CH₂ |
| **321** | 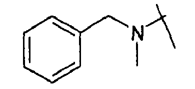 | CN | CH₂ |
| **322** | 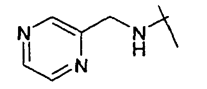 | CN | CH₂ |
| **323** | 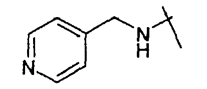 | CN | CH₂ |
| **324** | 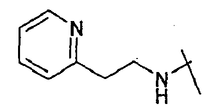 | CN | CH₂ |
| **325** | 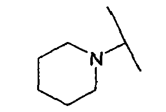 | CN | CH₂ |
| **326** | 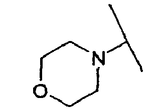 | CN | CH₂ |
| **327** | 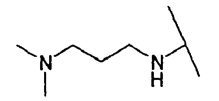 | H | CH₂ |
| **328** | 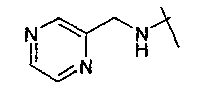 | H | CH₂ |
| **329** | 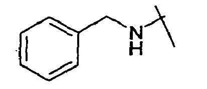 | H | S |
| **330** | 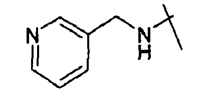 | H | S |
| **331** | 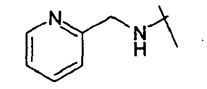 | H | S |
| **332** | 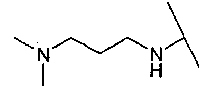 | H | S |
| **333** | 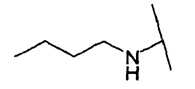 | CN | CH₂ |
| **334** | 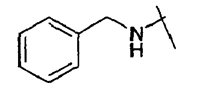 | CN | CH₂ |
| **335** | 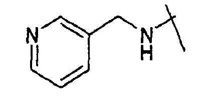 | CN | S |
| **336** | 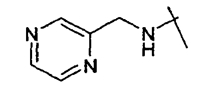 | CN | S |
| **337** | 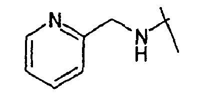 | CN | CH₂ |
| **338** | 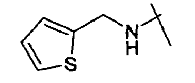 | CN | CH₂ |
| **339** | 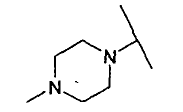 | CN | CH₂ |
| **340** | 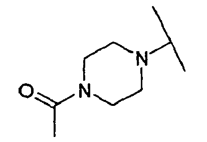 | CN | CH₂ |
| **341** | 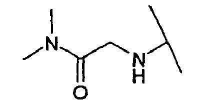 | H | S |
| **342** | 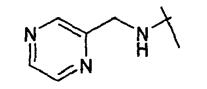 | H | S |
| **343** | 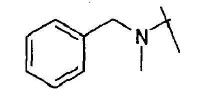 | H | S |
| **344** | 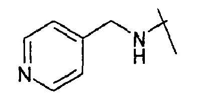 | H | S |
| **345** | 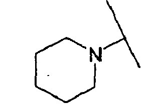 | H | S |
| **346** | 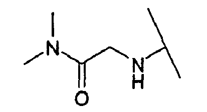 | H | S |
| **347** | 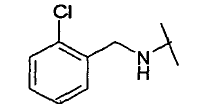 | H | S |
| **348** | 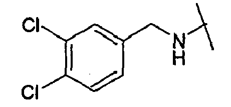 | H | S |
| **349** | 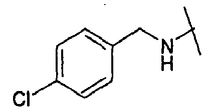 | H | S |
| **350** | 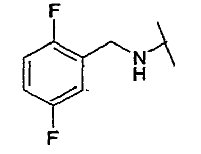 | H | S |
| **351** | 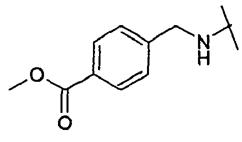 | H | S |
| **352** | 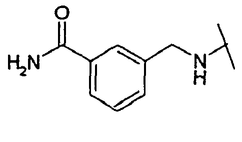 | H | S |
| **353** | 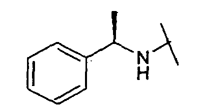 | H | S |
| **354** | 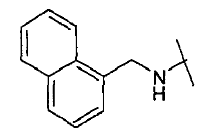 | H | S |
| **355** | 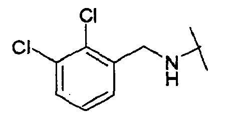 | H | S |
| **356** | 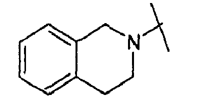 | H | S |
| **357** | 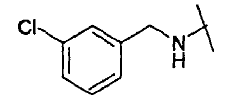 | H | S |
| **358** | 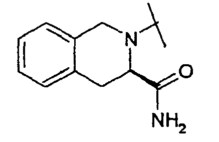 | H | S |
| **359** | 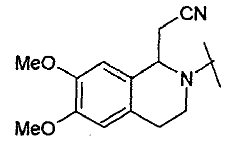 | H | S |
| **360** | 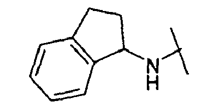 | H | S |
| **361** | 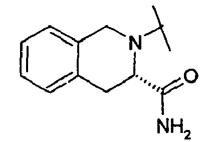 | H | CH₂ |
| **362** | 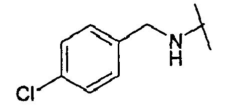 | H | CH₂ |
| **363** | 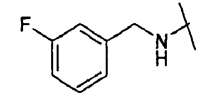 | H | S |
| **364** | 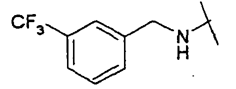 | H | S |
| **365** | 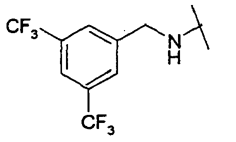 | H | S |
| **366** | 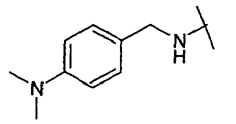 | H | S |
| **367** | 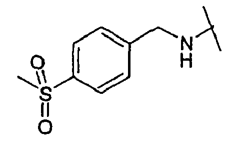 | H | S |
| **368** | 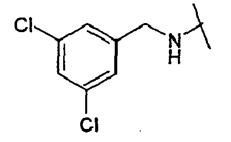 | H | S |
| **369** | 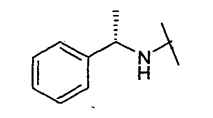 | H | S |
| **370** | 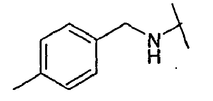 | H | S |
| **371** | 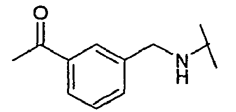 | H | S |
| **372** | 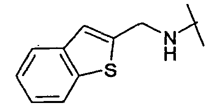 | H | S |
| **373** | 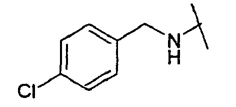 | H | S |
| **374** | 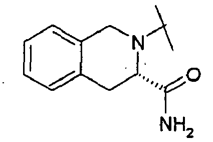 | H | S |
| **375** | 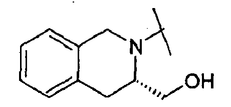 | H | S |
| **376** | 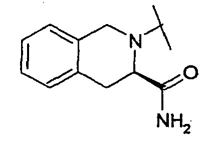 | H | CH₂ |
| **377** | 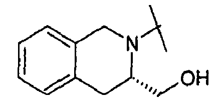 | H | CH₂ |
| **378** | 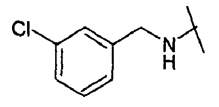 | H | CH₂ |

**TABLE 6 ― Examples 379 - 418**

| | | | | |
|---|---|---|---|---|
| 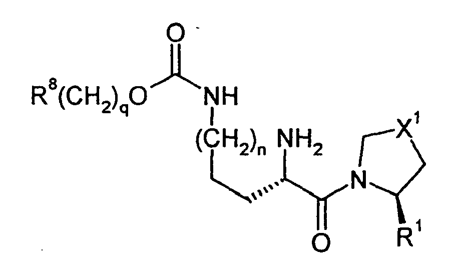 | | | | |

| **Example** | **n** | **R**^{**8**}**(CH**_{**2**}**)**_{**q**} | **R**^{**1**} | **X**^{**1**} |
|---|---|---|---|---|
| **379** | 1 | 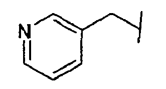 | CN | CH₂ |
| **380** | 2 | | CN | CH₂ |
| **381** | 1 | 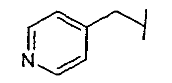 | CN | CH₂ |
| **382** | 2 | | CN | CH₂ |
| **383** | 1 | 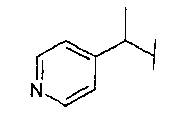 | CN | CH₂ |
| **384** | 2 | | CN | CH₂ |
| **385** | 1 | 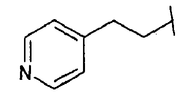 | CN | CH₂ |
| **386** | 2 | | CN | CH₂ |
| **387** | 1 | 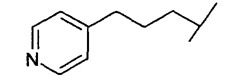 | CN | CH₂ |
| **388** | 2 | | CN | CH₂ |
| **389** | 1 | 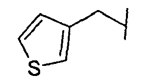 | CN | CH₂ |
| **390** | 2 | | CN | CH₂ |
| **391** | 1 | 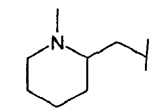 | CN | CH₂ |
| **392** | 2 | | CN | CH₂ |
| **393** | 1 | 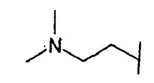 | CN | CH₂ |
| **394** | 2 | | CN | CH₂ |
| **395** | 1 | 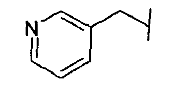 | H | CH₂ |
| **396** | 2 | | H | CH₂ |
| **397** | 1 | 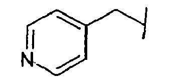 | H | S |
| **398** | 2 | | H | S |
| **399** | 1 | 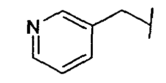 | CN | S |
| **400** | 2 | | CN | S |
| **401** | 1 | 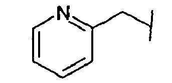 | CN | CH₂ |
| **402** | 2 | | CN | CH₂ |
| **403** | 1 | 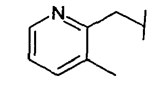 | CN | CH₂ |
| **404** | 2 | | CN | CH₂ |
| **405** | 1 | 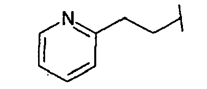 | CN | CH₂ |
| **406** | 2 | | CN | CH₂ |
| **407** | 1 | 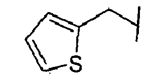 | CN | CH₂ |
| **408** | 2 | | CN | CH₂ |
| **409** | 1 | 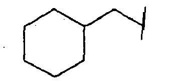 | CN | CH₂ |
| **410** | 2 | | CN | CH₂ |
| **411** | 1 | 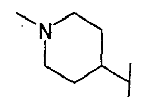 | CN | CH₂ |
| **412** | 2 | | CN | CH₂ |
| **413** | 1 | 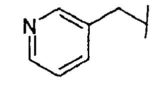 | H | S |
| **414** | 2 | | H | S |
| **415** | 1 | 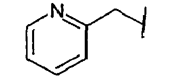 | H | S |
| **416** | 2 | | H | S |
| **417** | 1 | 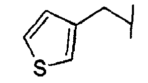 | H | S |
| **418** | 2 | | H | S |

**TABLE 7 - Examples 419 - 438**

| | | |
|---|---|---|
| 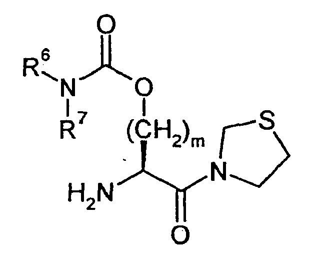 | | |

| **Example** | **m** | **R**^{**6**}**R**^{**7**}**N** |
|---|---|---|
| **419** | 1 | 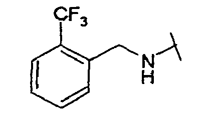 |
| **420** | 1 | 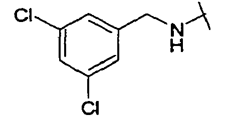 |
| **421** | 1 | 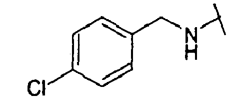 |
| **422** | 1 | 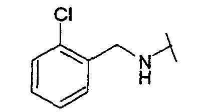 |
| **423** | 1 | 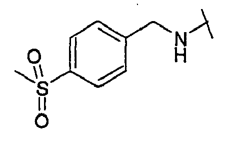 |
| **424** | 1 | 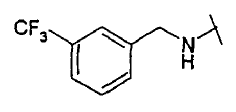 |
| **425** | 2 | |
| **426** | 3 | |
| **427** | 1 | 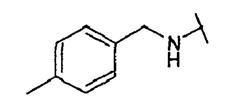 |
| **428** | 2 | |
| **429** | 3 | |
| **430** | 1 | 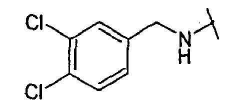 |
| **431** | 2 | |
| **432** | 3 | |
| **433** | 2 | 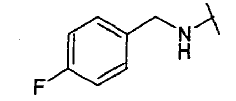 |
| **434** | 3 | |
| **435** | 2 | 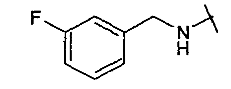 |
| **436** | 3 | |
| **437** | 2 | 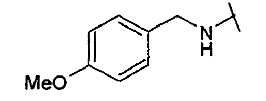 |
| **438** | 3 | |

**TABLE 8 - Examples 439 - 450**

| | | | | |
|---|---|---|---|---|
| 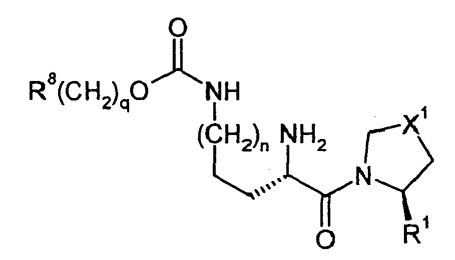 | | | | |
| **439** | 1 | 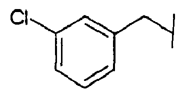 | CN | CH₂ |
| **440** | 2 | | CN | CH₂ |
| **441** | 1 | 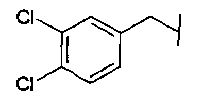 | CN | CH₂ |
| **442** | 2 | | CN | CH₂ |
| **443** | 1 | 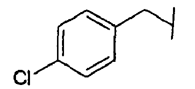 | H | S |
| **444** | 2 | | H | S |
| **445** | 1 | 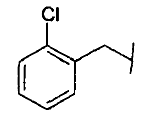 | CN | CH₂ |
| **446** | 2 | | CN | CH₂ |
| **447** | 1 | 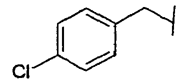 | CN | CH₂ |
| **448** | 2 | | CN | CH₂ |
| **449** | 1 | 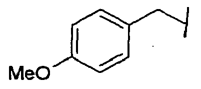 | CN | CH₂ |
| **450** | 2 | | CN | CH₂ |

### EXAMPLE 451

### Determination of activity

Compounds were assayed as inhibitors of DP-IV according to the methods described in WO95/15309. All the compounds described in the foregoing Examples were competitive inhibitors of DP-IV with Kᵢ values less than 300nM.

### EXAMPLE 452

### Determination of activity in vivo

The anti-diabetic action of selected compounds was demonstrated in Zucker obese rats using a standard oral glucose tolerance test. Control rats were given a solution of glucose by oral gavage, and plasma glucose levels were determined. These rats demonstrated a significant hyperglycaemia. Compounds according to the present invention were dissolved in glucose solution at various concentrations, such that the rats could be given varying doses of the compound simultaneously with the glucose challenge. The hyperglycaemic excursion was reduced in a dose-dependent manner in animals receiving between 0.1 and 100 mg/kg of DP-IV inhibitor.

### EXAMPLE 453

### Pharmaceutical formulation

Tablets containing 100mg of the compound of Example 1 as the active agent are prepared from the following:

| | |
|---|---|
| Compound of Example 1 | 200.0g |
| Corn starch | 71.0g |
| Hydroxypropylcellulose | 18.0g |
| Carboxymethylcellulose calcium | 13.0g |
| Magnesium stearate | 3.0g |
| Lactose | 195.0g |
| *Total* | *500.0g* |

The materials are blended and then pressed to give 2000 tablets of 250mg, each containing 100mg of the compound of Example 1.

The above demonstrates that the compounds according to the present invention are inhibitors of DP-IV and would accordingly be expected to be useful as therapeutic agents for the treatment of impaired glucose tolerance, type II diabetes, and other diseases where inhibition of this enzyme leads to an improvement in the underlying pathology or the symptoms.

The present invention is further defined in the following Claims.

## Claims

1. A compound selected from derivatives of formula 1, tautomers and stereoisomers thereof, and pharmaceutically acceptable salts of said derivatives, tautomers and isomers 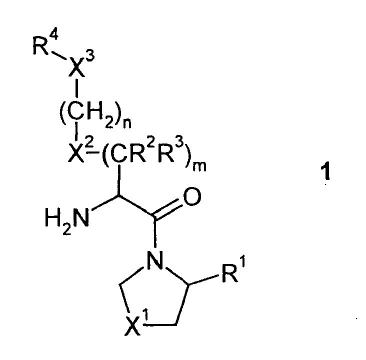 wherein:
X¹ is selected from a sulphur atom, an oxygen atom, a sulphinyl group, a sulphonyl group and a methylene group;
X² is selected from O, S and methylene;
X³ is either NR⁵ or a carbonyl or thiocarbonyl group;
R¹ is either a hydrogen atom or a nitrile group;
R² and R³ are independently selected from H and C₁-C₆ alkyl, or together may be -(CH₂)ₚ-;
R⁴ is R^{4A} when X³ is NR⁵ and R^{4B} when X³ is a carbonyl or thiocarbonyl group;
R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=O) and R⁸(CH₂)_{q}OC(=S);
R^{4B} is R⁶R⁷N;
R⁵ is H or C₁-C₆ alkyl;
R⁶ and R⁷ are selected independently from R⁸(CH₂)_{q} or together they are - (CH₂)₂-Z¹-(CH₂)₂- or -CHR⁹-Z²-CH₂-CHR¹⁰-;
R⁸ is selected from H, alkyl, benzo-fused cycloalkyl, acyl, dialkylcarbamoyl, dialkylamino, N-alkylpiperidyl, optionally substituted aryl, optionally substituted α-alkylbenzyl, optionally substituted aroyl, optionally substituted arylsulphonyl and optionally substituted heteroaryl;
R⁹ and R¹⁰ are selected independently from H, carbamoyl, hydroxymethyl and cyanomethyl;
Z¹ is selected from a covalent bond, -(CH₂)ᵣ-, -O-, -SOₜ- and -N((CH₂)_{q}R⁸)-;
Z² is an optionally substituted *ortho*-phenylene moiety;
m is 1 - 3;
n is 0 - 4;
p is 2 - 5;
q is 0 - 3;
r is 1 or 2; and
t is 0 - 2;
provided that when X² is CH₂, X³ is NH and R⁴ is R⁸CH₂O(CO) then R⁸ is not unsubstituted phenyl or nitrophenyl.

2. A compound according to Claim 1 wherein R¹ is a nitrile group.

3. A compound according to Claim 2 wherein the stereochemistry of the nitrile group is as shown in formula **2**

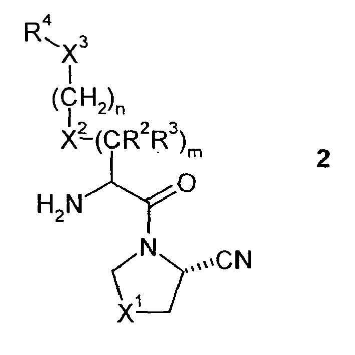

4. A compound according to Claim 1 or 2 wherein the stereochemistry of the centre adjacent to the primary amine is of the S configuration as shown in formula **3**

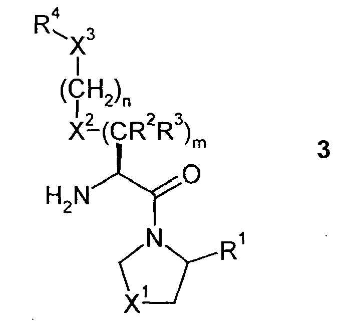

5. A compound according to Claim 4 wherein R¹ is a nitrile group and the stereochemistry of the nitrile group is as shown in formula **4**

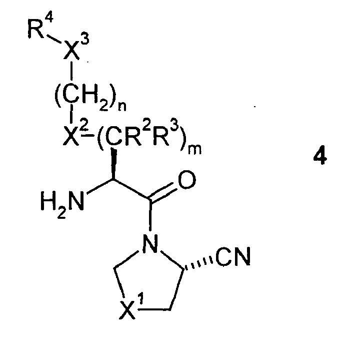

6. A compound according to any preceding Claim wherein m is 1.

7. A compound according to any preceding Claim wherein R² and R³ are independently H or methyl.

8. A compound according to any preceding Claim wherein m is 1, X² is -CH₂- and R² and R³ are both H.

9. A compound according to any of Claims 1 to 7 wherein m is 1, X² is -O- and one of R² and R³ is methyl and the other is H.

10. A compound according to any of Claims 1 to 7 wherein m is 1, X² is -S- and R² and R³ are both methyl.

11. A compound according to any preceding Claim wherein X³ is NH.

12. A compound according to Claim 11 wherein m is 1, R² and R³ are H and n is 1 or 2.

13. A compound according to Claim 12 wherein R⁴ is R⁸CO or R⁸NHCO and R⁸ is optionally substituted heteroaryl.

14. A compound according to any of Claims 1 to 7 wherein m is 1, X² is -CH₂-, R² and R³ are both H, n is 0 and X³ is CO.

15. A compound according to Claim 14 wherein R⁴ is R⁶NH.

16. A compound according to Claim 15 wherein R⁶ is optionally substituted heteroaryl.

17. A compound according to Claim 13 or Claim 16 wherein the heteroaryl group is unsubstituted or mono- or disubstituted and the substituents are selected from C₁-C₆ alkyl, C₁-C₆ alkyloxy, fluoro, chloro and trifluorornethyl groups.

18. A compound according to Claim 1 wherein R¹ is CN and X¹ is CH₂.

19. A compound according to Claim 1 wherein R¹ is H and X¹ is S.

20. A compound according to Claim 1 wherein X³ is NH and R⁴ is selected from R⁶R⁷N(CO), R⁸(CH₂)_{q}CO and R⁸(CH₂)_{q}SO₂.

21. A compound according to Claim 1 selected from:
(2*S*)-1-[*N*^{ω}-(Pyrazinyl-2-carbonyl)-L-ornithinyl]pyrrolidine-2-carbonitrile,
(2*S*)-1-[*N*^{ω}-(Pyrazinyl-2-carbonyl)-L-lysinyl]pyrrolidine-2-carbonitrile,
(2*S*)-1-[(2'*S*)-2'-Amino-4'-(pyrazinyl-2"-carbonylamino)butanoyl]pyrrolidine-2-carbonitrile,
(4*R*)-3-[*N*^{ω}-(Pyrazinyl-2-carbonyl)-L-lysinyl]thiazolidine-4-carbonitrile,
(2*S*)-1-[*N*^{ω}-(Pyridyl-3-methyl)-L-glutaminyl]pyrrolidine-2-carbonitrile,
1-[*N*^{ω}-(Pyrazinyl-2-carbonyl)-L-ornithinyl]pyrrolidine,
(2*S*)-1-[*S*-(Acetylaminomethyl)-L-cysteinyl]pyrrolidine-2-carbonitrile,
3-[*N*^{ω}-(2-Chloropyridyl-3-carbonyl)-L-ornithinyl]thiazolidine,
1-[*N*^{ω}-(2-Chloropyridyl-3-carbonyl)-L-ornithinyl]pyrrolidine,
(2S)-1-[*N*^{ω}-(2-Chloropyridyl-3-carbonyl)-L-ornithinyl]pyrrolidine-2-carbonitrile
3-[N^{ω}-(Pyrazinyl-2-carbonyl)-L-ornithinyl]thiazolidine,
3-[*N*^{ω}-(2-Quinoxaloyl)-L-lysinyl]thiazolidine,
3-[*N*^{ω}-(2-Quinoxaloyl)-L-omithinyl]thiazolidine,
(2S)-1-[*N*^{ω}-(2-Quinoxaloyl)-L-ornithinyl]pyrrolidine-2-carbonitrile,
3-[*N*^{ω}-(6-Methylpyrazinyl-2-carbonyl)-L-ornithinyl]thiazolidine,
3-[*N*^{ω}-(Isoquinoline-3-carbonyl)-L-ornithinyl]thiazolidine,
3-[*N*^{ω}-(6-Trifluoromethylnicotinoyl)-L-ornithinyl]thiazolidine,
(2*S*)-1-[(2'*R*)-3'-(Acetylaminomethylthio)-2'-amino-3'-methylbutanoyl]pyrrolidine-2-carbonitrile,
(2*S*)-1-[*S*-(3-Picolylcarbamoylmethyl)-L-cysteinyl]pyrrolidine-2-carbonitrile,
3-[*N*^{ω}-(3-Pyridyloxycarbonyl)-L-ornithinyl]thiazolidine,
3-[*O*-(3-Chlorobenzylcarbamoyl)serinyl]thiazolidine, and
3-[(2'*S*)-2'-Amino-5'-oxo-5'-(1",2",3",4"-tetrahydroisoquinolin-2"-yl)pentanoyl]-thiazolidine.

22. A pharmaceutical composition for human therapeutic use comprising at least one compound according to any preceding Claim.

23. A composition for the treatment of at least one of type 2 diabetes, impaired glucose tolerance, growth hormone deficiency, polycystic ovary syndrome, and auto-immune and inflammatory diseases, the composition comprising at least one compound which is selected from derivatives of formula A, tautomers and stereoisomers thereof, and pharmaceutically acceptable salts of said derivatives, tautomers and isomers, or which is according to any of claims 2 to 21 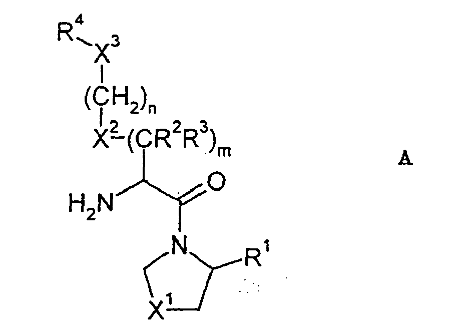 wherein:
X¹ is selected from a sulphur atom, an oxygen atom, a sulphinyl group, a sulphonyl group and a methylene group;
X² is selected from O, S and methylene;
X³ is either NR⁵ or a carbonyl or thiocarbonyl group;
R¹ is either a hydrogen atom or a nitrile group;
R² and R³ are independently selected from H and C₁-C₆ alkyl, or together may be -(CH₂)ₚ-;
R⁴ is R^{4A} when X³ is NR⁵ and R^{4B} when X³ is a carbonyl or thiocarbonyl group;
R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=O) and R⁸(CH₂)_{q}OC(=S);
R^{4B} is R⁶R⁷N;
R⁵ is H or C₁-C₆ alkyl;
R⁶ and R⁷ are selected independently from R⁸(CH₂)_{q} or together they are - (CH₂)₂-Z¹-(CH₂)₂- or -CHR⁹-Z²-CH₂-CHR¹⁰-;
R⁸ is selected from H, alkyl, benzo-fused cycloalkyl, acyl, dialkylcarbamoyl, dialkylamino, N-alkylpiperidyl, optionally substituted aryl, optionally substituted α-alkylbenzyl, optionally substituted aroyl, optionally substituted arylsulphonyl and optionally substituted heteroaryl;
R⁹ and R¹⁰ are selected independently from H, carbamoyl, hydroxymethyl and cyanomethyl;
Z¹ is selected from a covalent bond, -(CH₂)ᵣ-, -O-, -SOₜ- and -N((CH₂)_{q}R⁸)-;
Z² is an optionally substituted *ortho*-phenylene moiety;
m is 1 - 3;
n is 0 - 4;
p is 2-5;
q is 0 - 3;
r is 1 or 2; and
t is 0 - 2.

24. The use of a compound for the preparation of a pharmaceutical composition for the treatment of at least one of type 2 diabetes, impaired glucose tolerance, growth hormone deficiency, polycystic ovary syndrome, and auto-immune and inflammatory diseases, the compound being selected from derivatives of formula A, tautomers and stereoisomers thereof, and pharmaceutically acceptable salts of said derivatives, tautomers and isomers, or being according to any of claims 2 to 21 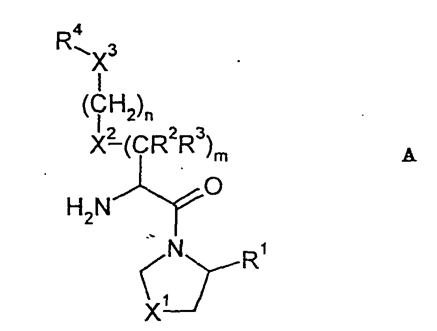 wherein:
X¹ is selected from a sulphur atom, an oxygen atom, a sulphinyl group, a sulphonyl group and a methylene group;
X² is selected from O, S and methylene;
X³ is either NR⁵ or a carbonyl or thiocarbonyl group;
R¹ is either a hydrogen atom or a nitrile group;
R² and R³ are independently selected from H and C₁-C₆ alkyl, or together may be -(CH₂)ₚ-;
R⁴ is R^{4A} when X³ is NR⁵ and R^{4B} when X³ is a carbonyl or thiocarbonyl group;
R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=O) and R⁸(CH₂)_{q}OC(=S);
R^{4B} is R⁶R⁷N;
R⁵ is H or C₁-C₆ alkyl;
R⁶ and R⁷ are selected independently from R⁸(CH₂)_{q} or together they are
- (CH₂)₂-Z¹-(CH₂)₂- or -CHR⁹-Z²-CH₂-CHR¹⁰-;
R⁸ is selected from H, alkyl, benzo-fused cycloalkyl, acyl, dialkylcarbamoyl, dialkylamino, N-alkylpiperidyl, optionally substituted aryl, optionally substituted α-alkylbenzyl, optionally substituted aroyl, optionally substituted arylsulphonyl and optionally substituted heteroaryl;
R⁹ and R¹⁰ are selected independently from H, carbamoyl, hydroxymethyl and cyanomethyl;
Z¹ is selected from a covalent bond, -(CH₂)ᵣ-, -O-, -SOₜ- and -N((CH₂)_{q}R⁸)-;
Z² is an optionally substituted *ortho*-phenylene moiety;
m is 1 - 3;
n is 0 - 4;
p is 2 - 5;
q is 0 - 3;
r is 1 or 2; and
t is 0 - 2.

25. The use of a compound for the treatment of type 2 diabetes, impaired glucose tolerance, growth hormone deficiency, polycystic ovary syndrome, or auto-immune or inflammatory disease, the compound being selected from derivatives of formula A, tautomers and stereoisomers thereof, and pharmaceutically acceptable salts of said derivatives, tautomers and isomers, or being according to any of claims 2 to 21 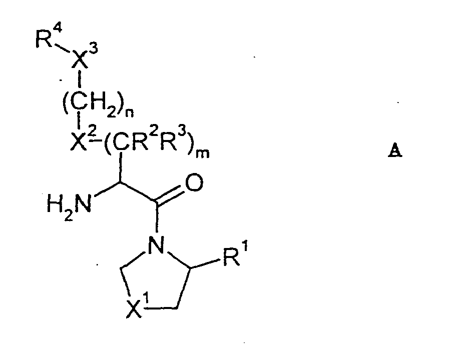 wherein :
X¹ is selected from a sulphur atom, an oxygen atom, a sulphinyl group, a sulphonyl group and a methylene group;
X² is selected from O, S and methylene;
X³ is either NR⁵ or a carbonyl or thiocarbonyl group;
R¹ is either a hydrogen atom or a nitrile group;
R² and R³ are independently selected from H and C₁-C₆ alkyl, or together may be -(CH₂)ₚ-;
R⁴ is R^{4A} when X³ is NR⁵ and R^{4B} when X³ is a carbonyl or thiocarbonyl group;
R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=O) and R⁸(CH₂)_{q}OC(=S);
R^{4B} is R⁶R⁷N;
R⁵ is H or C₁-C₆ alkyl;
R⁶ and R⁷ are selected independently from R⁸(CH₂)_{q} or together they are
- (CH₂)₂-Z¹-(CH₂)₂- or -CHR⁹-Z²-CH₂-CHR¹⁰-;
R⁸ is selected from H, alkyl, benzo-fused cycloalkyl, acyl, dialkylcarbamoyl, dialkylamino, N-alkylpiperidyl, optionally substituted aryl, optionally substituted α-alkylbenzyl, optionally substituted aroyl, optionally substituted arylsulphonyl and optionally substituted heteroaryl;
R⁹ and R¹⁰ are selected independently from H, carbamoyl, hydroxymethyl and cyanomethyl;
Z¹ is selected from a covalent bond, -(CH₂)ᵣ-, -O-, -SOₜ- and -N((CH₂)_{q}R⁸)-;
Z² is an optionally substituted *ortho*-phenylene moiety;
m is 1 - 3;
n is 0 - 4;
p is 2 - 5;
q is 0 - 3;
r is 1 or 2; and
t is 0 - 2.

26. A method of treatment of at least one of type 2 diabetes, impaired glucose tolerance, growth hormone deficiency, polycystic ovary syndrome, and auto-immune and inflammatory diseases, which comprises the administration to a patient in need of such treatment of a therapeutically effective amount of at least one compound which is selected from derivatives of formula A, tautomers and stereoisomers thereof, and pharmaceutically acceptable salts of said derivatives, tautomers and isomers, or which is according to any of claims 2 to 21 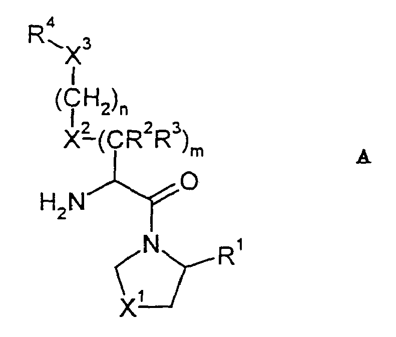 wherein:
X¹ is selected from a sulphur atom, an oxygen atom, a sulphinyl group, a sulphonyl group and a methylene group;
X² is selected from O, S and methylene;
X³ is either NR⁵ or a carbonyl or thiocarbonyl group;
R¹ is either a hydrogen atom or a nitrile group;
R² and R³ are independently selected from H and C₁-C₆ alkyl, or together may be -(CH₂)ₚ-;
R⁴ is R^{4A} when X³ is NR⁵ and R^{4B} when X³ is a carbonyl or thiocarbonyl group;
R^{4A} is selected from R⁶R⁷NC(=O), R⁶R⁷NC(=S); R⁸(CH₂)_{q}C(=O), R⁸(CH₂)_{q}C(=S), R⁸(CH₂)_{q}SO₂, R⁸(CH₂)_{q}OC(=O) and R⁸(CH₂)_{q}OC(=S);
R^{4B} is R⁶R⁷N;
R⁵ is H or C₁-C₆ alkyl;
R⁶ and R⁷ are selected independently from R⁸(CH₂)_{q} or together they are - (CH₂)₂-Z¹-(CH₂)₂- or -CHR⁹-Z²-CH₂-CHR¹⁰-;
R⁸ is selected from H, alkyl, benzo-fused cycloalkyl, acyl, dialkylcarbamoyl, dialkylamino, N-alkylpiperidyl, optionally substituted aryl, optionally substituted α-alkylbenzyl, optionally substituted aroyl, optionally substituted arylsulphonyl and optionally substituted heteroaryl;
R⁹ and R¹⁰ are selected independently from H, carbamoyl, hydroxymethyl and cyanomethyl;
Z¹ is selected from a covalent bond, -(CH₂)ᵣ-, -O-, -SOₜ- and -N((CH₂)_{q}R⁸)-;
Z² is an optionally substituted *ortho*-phenylene moiety;
m is 1 - 3;
n is 0 - 4;
pis2-5;
q is 0 - 3;
r is 1 or 2; and
t is 0 - 2.
